# EUROPEAN PATENT APPLICATION

(11) **EP 4 148 128 A1**
(43) Date of publication of application: **15.03.2023**
(21) Application number: 21195842.6
(22) Date of filing: 09.09.2021
(51) Int. Cl.: C12N 9/22, A61K 38/45, A61K 48/00, C12N 15/90

(54) **POLYPEPTIDES WITH TRANSPOSITIONAL ACTIVITY**

(71) Applicant: Paul-Ehrlich-Institut, Bundesinstitut für Impfstoffe und biomedizinische Arzneimittel, 63225 Langen (DE)
(72) Inventor: Ivics, Zoltan, 13125 Berlin (DE); Kesselring, Lisa, 85567 Grafing bei München (DE); Miskey, Csaba, 63225 Langen (DE)
(74) Representative: Moré, Solveig Helga

(57) **Abstract**

The present invention relates to polypeptides with transpositional activity, particularly engineered polypeptides with transpositional activity, nucleic acids encoding such polypeptides, vectors comprising said nucleic acids, a cell comprising said nucleic acid, methods of integrating an exogenous nucleic acid into the genome using said polypeptides, said polypeptides for use in medicine and/or several gene therapies; and a pharmaceutical composition comprising said polypeptides, nucleic acids, vectors or cells. Particularly, the present invention relates to said polypeptide, which is engineered to have a gain of specificity of integration into the genome.

## Description

The present invention relates to polypeptides with transpositional activity, particularly engineered polypeptides with transpositional activity, nucleic acids encoding such polypeptides, vectors comprising said nucleic acids, a cell comprising said nucleic acid, methods of integrating an exogenous nucleic acid into the genome using said polypeptides, said polypeptides for use in medicine and/or several gene therapies; and a pharmaceutical composition comprising said polypeptides, nucleic acids, vectors or cells. Particularly, the present invention relates to said polypeptide, which is engineered to have a gain of specificity of integration into the genome.

### Background of the Invention

Polypeptides with transposase activity are a popular tool for therapeutic genome engineering. Transposons or transposable elements include a (short) nucleic acid sequence with terminal repeat sequences upstream and downstream thereof. Active transposons encode enzymes that facilitate the excision and insertion of the nucleic acid into target DNA sequences.

The *Sleeping Beauty* (SB, a Tc1/*mariner* superfamily transposon) transposon is one of the known tools, which is used for therapeutic genome engineering. This exemplary tool has been investigated in the past and in particular a variant was developed which shows hyperactivity, resulting in an enhanced efficient insertion of transposons of varying size into the nucleic acid of a cell or the insertion of DNA into the genome of a cell thus allowing more efficient transcription/translation than known transposons.

SB is a synthetic transposon that was reconstructed based on sequences of transpositionally inactive elements isolated from fish genomes. SB is the most thoroughly studied vertebrate transposon to date, and it supports a full spectrum of genetic engineering applications, including the generation of transgenic cell lines, induced pluripotent stem cell (iPSC) reprogramming, phenotype-driven insertional mutagenesis screens in the area of cancer biology, germline gene transfer in experimental animals and somatic gene therapy both *ex vivo* and *in vivo.* On the genomic scale, SB transposons exhibit a close-to-random integration profile with a slight bias towards integration into genes and their upstream regulatory sequences in cultured mammalian cell lines. On a local scale, SB preferentially inserts into TA dinucleotides (like *Mos1*), and shows additional target site preferences based on physical properties of the DNA, including bendability, A-philicity and a symmetrical pattern of hydrogen bonding sites in the major groove of the tDNA. However, random integration into the genome carries a certain genotoxic risk in human applications. Thus, there is a need of a transposon system, wherein integration is secured and insertion into the genome does not pose a risk with respect to oncogenic transformation, or at least wherein the risk is reduced.

The present inventors have unexpectedly found specific transposase variants, which exhibit *inter alia*: a gain of specificity of integration into the genome, in particular into palindromic AT repeat target sequence. The inventors demonstrate enhanced DNA bendability of target sites enriched in non-nucleosomal DNA by the discovered variants. The variant were further found to de-target exons as well as transcriptional regulatory regions of genes in the human genome, thus enhancing safety and utility of the variants in gene therapy applications.

### Summary of the Invention

In a first aspect, the present invention provides a polypeptide with transposase activity comprising a variant of a naturally occurring transposase containing the following secondary structural elements: α1 helix- α2 helix- β1 sheet- β2 sheet- β3 sheet-β4 sheet- β5 sheet-α3 helix-β6 sheet- η1-α4 helix-η2-α5 helix- α6 helix-α7 helix-α8 helix,
wherein
(i) a naturally occurring H in the loop connecting the β3 sheet and the β4 sheet is substituted with V, A, N, C, Q, G, I, L, M, F, P, R, S, T, W, K or Y, preferably with V or P, or
   a naturally occurring F in the loop connecting the β3 sheet and the β4 sheet is substituted with V, A, N, C, Q, G, I, L, M, P, R, S, T, W, K, H or Y, preferably with V or P, or
   a naturally occurring Y in the loop connecting the β3 sheet and the β4 sheet is substituted with V, A, N, C, Q, G, I, L, M, F, P, R, S, T, W, H or K, preferably with V or P, or
   a naturally occurring L in the loop connecting the β3 sheet and the β4 sheet is substituted with V, A, N, C, Q, G, I, M, F, P, R, S, T, W, H, Y or K, preferably with V or P, or
   a naturally occurring S in the loop connecting the β3 sheet and the β4 sheet is substituted with V, A, N, C, Q, G, I, L, M, F, P, R, T, W, H, Y or K, preferably with V or P;
      and/or
(ii) at least one naturally occurring Q, P, S, or T in η1 connecting the β6 sheet with α4 helix is substituted with R, S or A, preferably with R;
   and/or
(iii) at least one naturally occurring K, I, S, T, V, P or C in η1 connecting the β6 sheet with α4 helix is preferably substituted with R;
wherein the variant of the naturally occurring transposase comprises at least one of the substations (i), (ii) or (iii).

In a second aspect, the invention relates to a nucleic acid comprising a nucleic acid sequence encoding a polypeptide of the first aspect.

In a third aspect, the invention relates to a vector comprising the nucleic acid molecule of the second aspect.

In a fourth aspect, the invention relates to a cell comprising the nucleic acid of the second aspect or the vector of the third aspect.

In a fifth aspect, the invention relates to an *in vitro* method of integrating an exogenous nucleic acid into the genome of a cell, the method comprising the steps of:
a) providing an isolated cell;
b) providing in the cell a polypeptide of any of claims 1 to 6; and
c) providing in the cell a nucleic acid comprising the exogenous nucleic acid or a vector comprising the same.

In a sixth aspect, the invention relates to an *in vivo* method of integrating an exogenous nucleic acid into the genome of a cell of a subject, the method comprising the step of administering the polypeptide, the nucleic acid, or the vector of any of the afore mentioned aspects of the invention and a nucleic acid comprising the exogenous nucleic acid or a vector comprising said nucleic acid to the subject.

In another aspect, the invention relates to the mentioned polypeptide, nucleic acid, vector, or cell for use in medicine, in particular in gene therapy.

In a further aspect, the invention relates to a pharmaceutical composition comprising the mentioned polypeptide, nucleic acid, vector or cell and a pharmaceutically acceptable carrier, adjuvant or vehicle.

### List of Figures

In the following, the content of the figures comprised in this specification is described. In this context please also refer to the detailed description of the invention above and/or below.

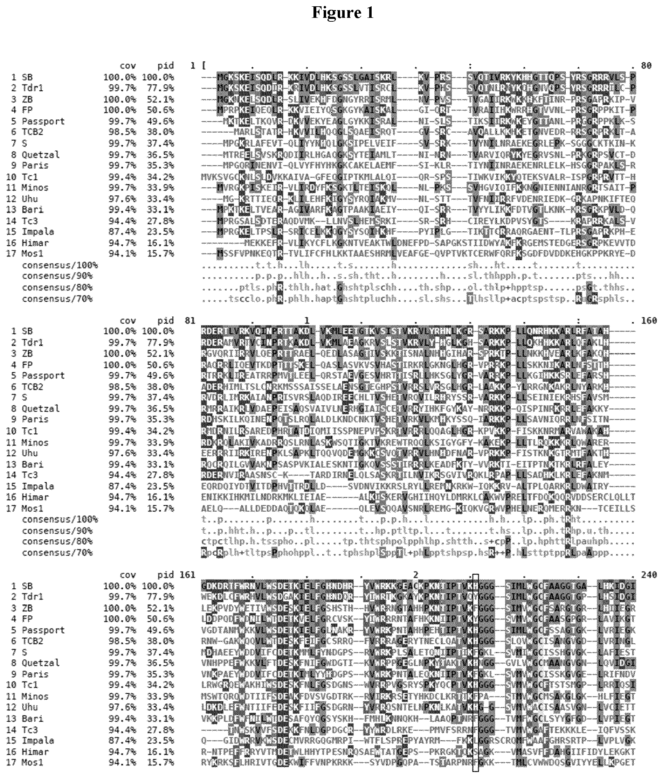

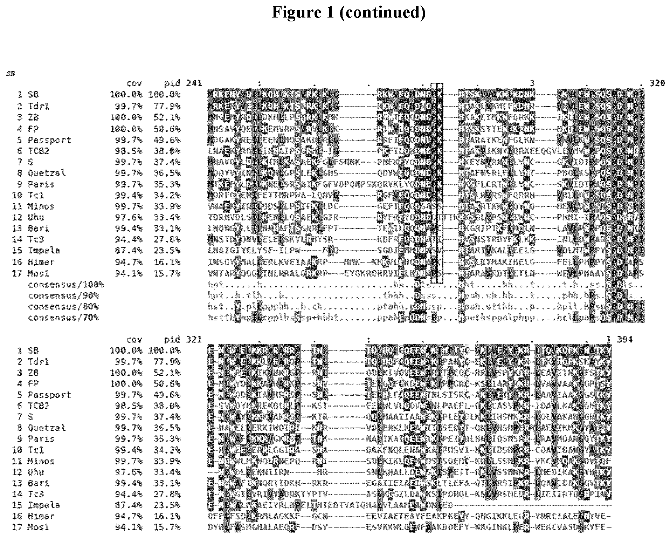

- **Fig. 1:**: **Alignment of the amino acid sequences of exemplary transposase of the Tc1/mariner superfamily transposases.** All amino acids corresponding to amino acid position 187, 247 and 248 of SB are highlighted with a box. Fig. 1 thus allows the skilled person to determine the amino acid position within all of the other aligned transposases that correspond to amino acid position 187, 247 and 248, respectively, and which could be substituted with a different amino acid in the same way as outlined exemplary for SB. Fig. 1 only depicts an alignment of a selection of transposase of the Tc1/mariner superfamily transposases. Further transposases of this superfamily could be added to the alignment allowing the identification of amino acids corresponding to amino acid position 187, 247 and 248 of SB in these transposases. The transposases shown belong to the group of transposases with known transposase activity. The amino acid sequence of SB (SEQ ID NO: 1) was used as the reference amino acid sequence in this alignment.

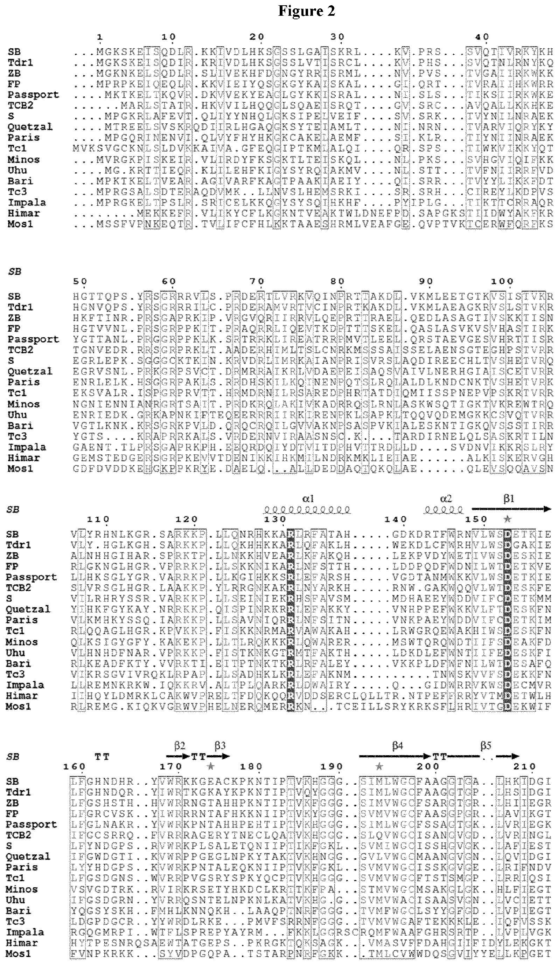

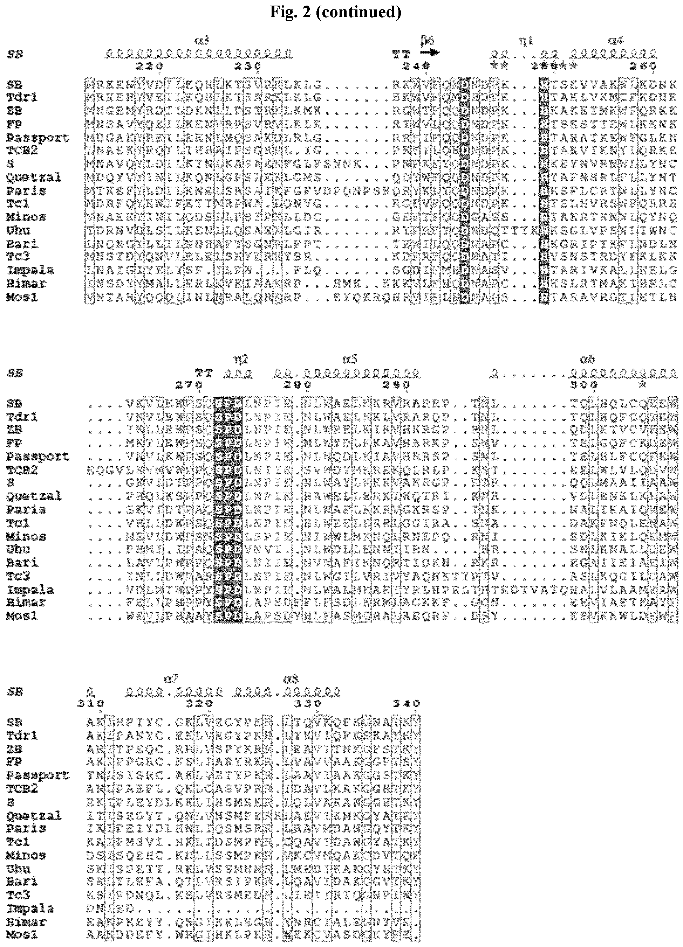

- **Fig.2:**: **Alignment of the amino acid sequences of transposase that are close relatives of Sleeping Beauty transposase.** All amino acids corresponding to amino acid position 187, 247 and 248 of SB are highlighted with a box. Fig. 1 thus allows the skilled person to determine the amino acid position within all of the other aligned transposases that correspond to amino acid position 187, 247 and 248, respectively, and which could be substituted with a different amino acid in the same way as outlined exemplary for SB. Fig. 2 only depicts an alignment of a selection of some transpositionally active members of the Tc1/mariner transposase superfamily. Further transposases could be added to the alignment allowing the identification of amino acids corresponding to amino acid position 187, 247 and 248 of SB in these transposases. On top of the sequence alignment the secondary structures α1 helix- α2 helix- β1 sheet- β2 sheet- β3 sheet-β4 sheet- β5 sheet-α3 helix-β6 sheet- η1-α4 helix-η2-α5 helix- α6 helix-α7 helix-α8 helix belonging to the corresponding amino acid stretches are indicated. This further allows the skilled person to determine the amino acid position within all of the other aligned transposases that correspond to specific amino acids in η1 connecting the β6 sheet with α4 helix, or connecting the β3 sheet and the β4 sheet. On the same way further transposases of this superfamily could be added to the alignment allowing the identification of amino acids corresponding to said positions in η1 connecting the β6 sheet with α4 helix, or connecting the β3 sheet and the β4 sheet. The amino acid sequence of SB (SEQ ID NO: 1) was used as the reference amino acid sequence in this alignment.

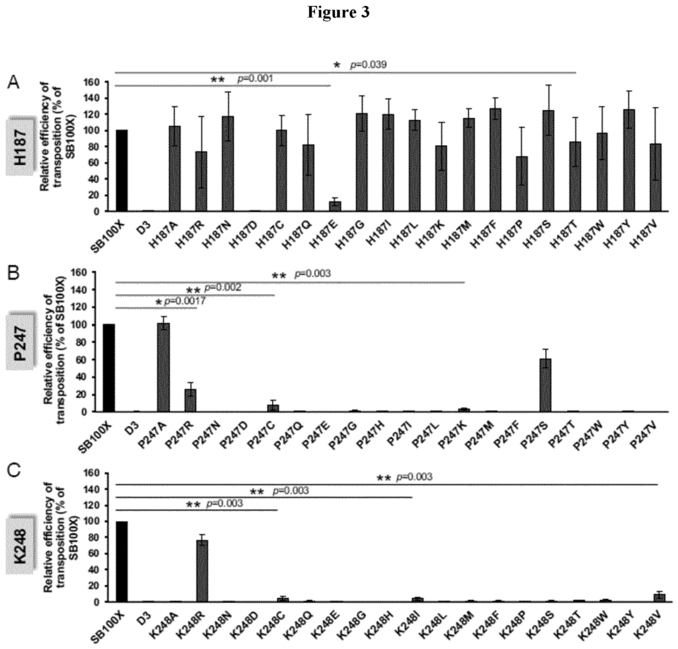

- **Fig. 3:**: **Transpositional activity of *Sleeping Beauty* transposase mutants.** Relative transpositional activities for H187 **(A),** P247 **(B)** and K248 **(C).** Plasmids expressing transposase mutants were transiently cotransfected with a transposondonor plasmid (pT2B/puro) into HeLa cells. Cells were selected for puromycin resistance and stained with methylene blue to identify viable cell colonies. Colony numbers were normalized to the SB100X positive control, whose transposition efficiency was set to 100%. An inactive SB transposase (D3) was included as negative control. Data are represented as mean ±SD, n=3 biological replicates. Differences in transposition activities are significant as determined by Student's t-test for the indicated mutants.

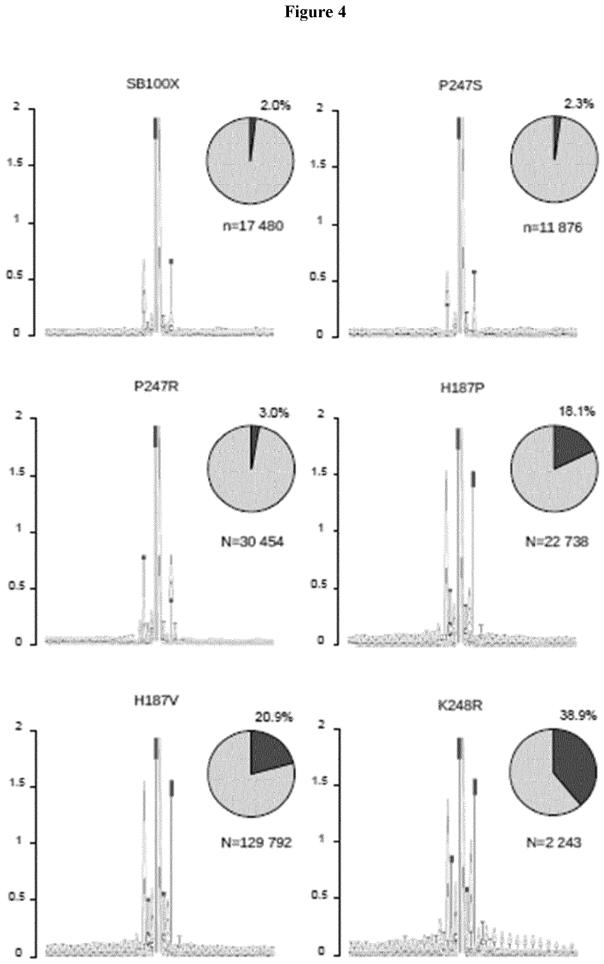

- **Fig. 4:**: **Integration sites of the transposase mutants.** The sequence logos show the majority-rule consensus sequences at the genomic insertion loci in a 60-bp window, centered around the target TA dinucleotides. The value 2 (log24) on the *y* axis stands for maximum possible frequency. The pie charts depict the percentages of insertions occurring at the SB-specific ATATATAT consensus motif. The N values represent the numbers of uniquely mappable SB transposon insertions.

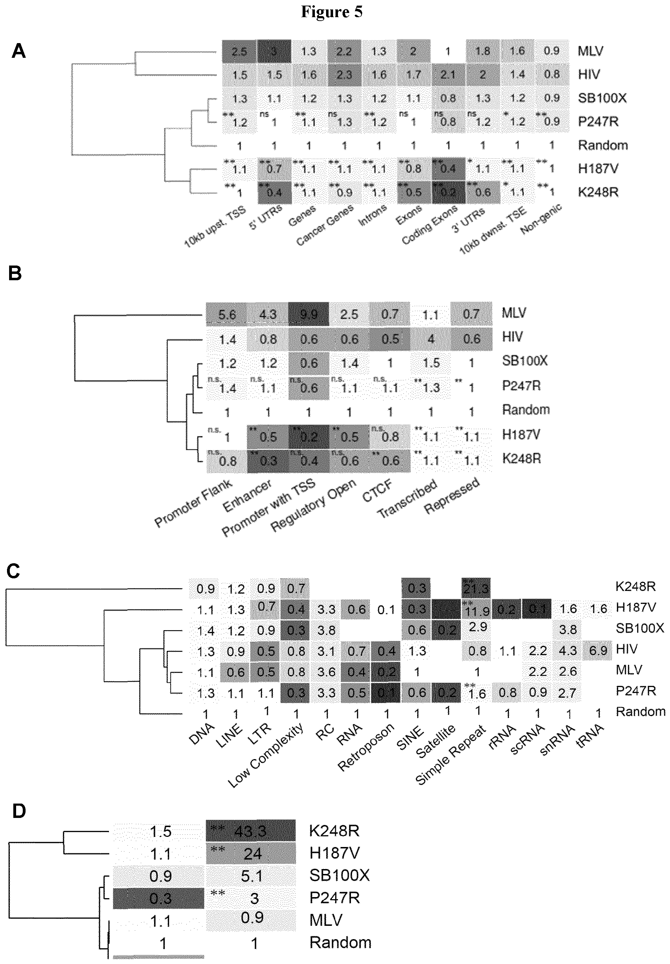

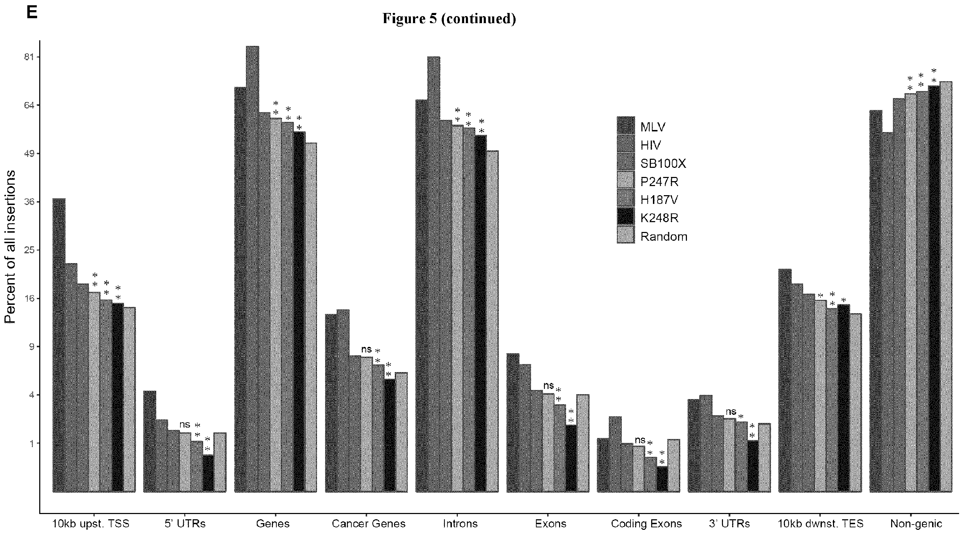

- **Fig. 5:**: **Representation of insertion sites in genomic features and chromatin defined functional segments. (A)** Integration loci of MLV, HIV, SB100X and its mutant derivatives were counted in gene-related segments of the human genome. The numbers represent fold change increase (red) or decrease (blue) in insertion frequencies compared to a theoretical random control (set to 1). The dendogram on the left is based on the mean frequency values of the rows. Statistical significance (Fisher exact test) measured for the values of the K248R, P247R and H187V mutants *vs.* SB100X is indicated by stars; * *p*<0.05, ** *p*<0.001, ns: not significant. **(B)** Representation of insertion sites in functional genomic segments as defined by epigenetic signal patterns. Color code, dendogram and statistical significance as indicated for panel **(A).** The enrichment in simple repeats integrations catalyzed by H187V and K248R is shown in panel **(C)** and the preference of integration into ATATATAT, the H187V variant is shown in panel **(D).** The overall percentages of insertions into these genomic regions are presented in **(E).**

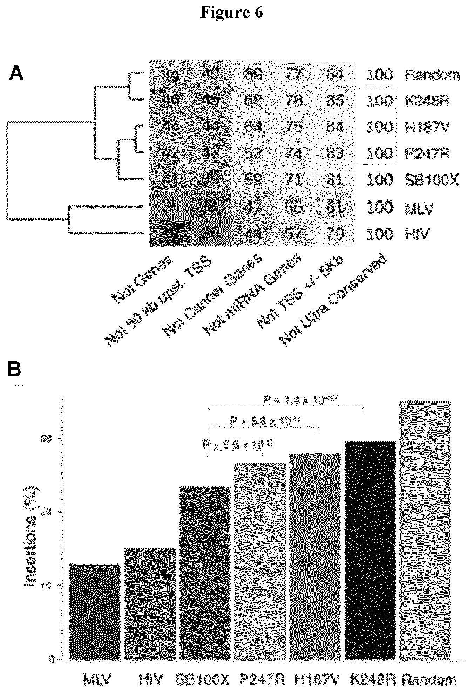

- **Fig. 6:**: **Insertion frequencies in genomic safe harbors. (A)** The numbers indicate the percentage representation of insertions in the safe harbor sub-categories listed below the table. The darker the color the lower the frequency of insertion in a given safe harbor category, opposed to the ideal case of 100%. ** *p*<0.001, Fischer test as compared to SB100X. **(B)** Overall representation of insertions in genomic safe harbors.

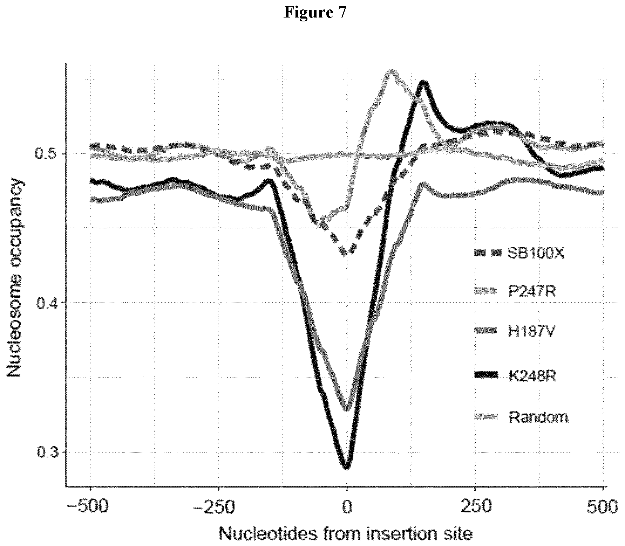

- **Fig. 7:**: **Nucleosome occupancy at transposon insertion sites.** Relative frequencies of insertions by SB100X and the K248R, P247R and H187V mutants into sites associated with nucleosomes as determined by MNase-Seq data on human HepG2 cells. The frequencies are depicted in a window that extends 500 bp upstream and 500 bp downstream from the transposon integration sites.

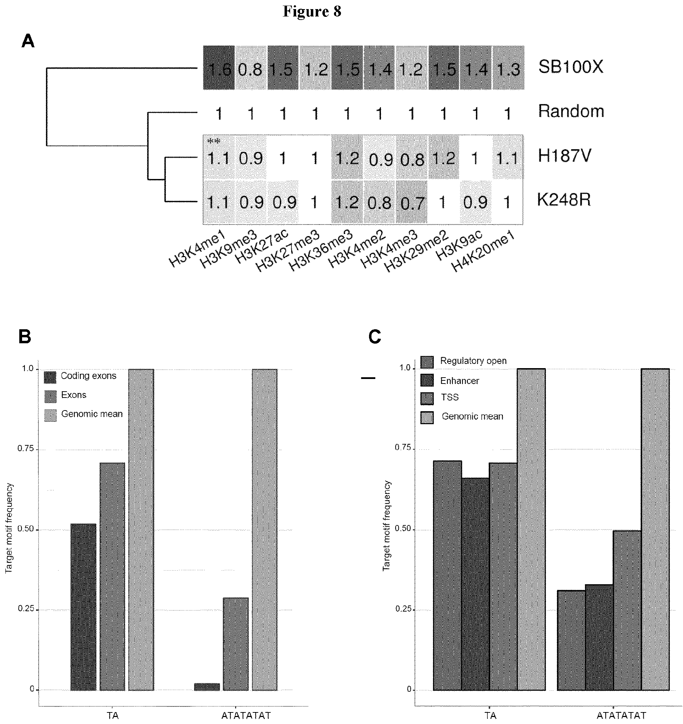

- **Fig. 8:**: **H187, P247 and K248 transposase mutants preferably target TA rich genomic regions.** Panel (A) shows that H187V and K248R insertions are generally depleted in histone modifications. Exons, in particular coding exons, are relatively poor in TA dinucleotides (Fig. 8B). However, exons are the preferred target sites for SB transposition.

### Detailed Description of the Invention

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Klbl, H. eds. (1995), Helvetica Chimica Acta, CH-4010 Basel, Switzerland) and as described in "Pharmaceutical Substances: Syntheses, Patents, Applications" by Axel Kleemann and Jurgen Engel, Thieme Medical Publishing, 1999; the "Merck Index: An Encyclopedia of Chemicals, Drugs, and Biologicals", edited by Susan Budavari et al., CRC Press, 1996, and the United States Pharmacopeia-25/National Formulary-20, published by the United States Pharmcopeial Convention, Inc., Rockville Md., 2001.

The practice of the present invention will employ, unless otherwise indicated, conventional methods of chemistry, biochemistry, cell biology, immunology. and recombinant DNA techniques which are explained in the literature in the field.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. In the following passages, different aspects of the invention are defined in more detail. Each aspect so defined may be combined with any other aspect or aspects unless clearly indicated to the contrary. In particular, any feature indicated as being optional, preferred or advantageous may be combined with any other feature or features indicated as being optional, preferred or advantageous.

In the following, the elements of the present invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

### Definitions

In the following, some definitions of terms frequently used in this specification are provided. These terms will, in each instance of its use, in the remainder of the specification have the respectively defined meaning and preferred meanings.

As used in this specification and the appended claims, the singular forms "a", "an", and "the" include plural referents, unless the content clearly dictates otherwise.

The term "transposase" as used herein refers to an enzyme that is a component of a functional nucleic acid-protein complex capable of transposition and which is mediating transposition. The term "transposase" also refers to integrases from retrotransposons or of retroviral origin. A "transposition reaction" as used herein refers to a reaction where a transposon inserts into a target nucleic acid. Primary components in a transposition reaction are a transposon and a transposase or an integrase enzyme.

The terms "naturally occurring transposase" or "wild-type transposase" are used interchangeably in the context of the present invention as those unmodified amino acid sequences of the transposases that have been isolated from a naturally occurring species. Such amino acid sequences are readily available at EBI or NCBI.

The term "transpositional activity" as used herein refers to the of a given transposase can be assessed in a transposition reaction. Suitable experimental set-ups are described in the experimental section herein or may use the classical binary transposition assay as described in Ivics, 1997 Cell 91: 501-510.

The term "transposase" refers to a continuous stretch of amino acids that has 100% of the transpositional activity of a full length naturally occurring transposase. A transposase may lack 1 to 10 N-and/or C-terminal amino acid sequences of the full length naturally occurring transposase. Preferably, it may lack a methionine at position M1, if the transposase is comprised in a fusion protein, which comprises another protein at the N-terminus of the transposase.

The term "substitution" as used in the context of the present invention refers to the exchange of one nucleotide or amino acid with one nucleotide in a nucleic acid sequence and with one amino acid in an amino acid sequence, respectively.

The term "consensus sequence" as used in the context of the present invention refers to a calculated order of most frequent residues, either nucleotide or amino acid, found at each position in a sequence alignment between two or more sequences. It represents the results of a multiple sequence alignment in which related sequences are compared to each other and similar sequence motifs are calculated. Conserved sequence motifs are depicted as consensus sequences, which indicate identical amino acids, i.e. amino acids identical among the compared sequences, conserved amino acids, i.e. amino acids which vary among the compared amino acid sequence but wherein all amino acids belong to a certain functional or structural group of amino acids, e.g. polar or neutral, and variable amino acids, i.e. amino acids which show no apparent relatedness among the compared sequence. Fig. 1 depicts an alignment of sequences of transposases including some transpositionally active members of the Tc1/mariner transposase superfamily, and below the alignment the derived consensus sequence. The alignment algorithm used to generate the alignments mentioned was CLUSTAL Omega using the following settings: dealign input sequences: no, mbed-like clustering guide-tree: yes, mbed-like clustering iteration: yes, number of combined iterations: default, max guide tree iterations: default, max hmm iterations: default. Amino acid conservation is highlighted in MView. Fig. 2 depicts the same alignment as in Fig. 1, but with a secondary structure information obtained for the catalytic domain of the SB transposase (PDB entry code: 5CR4) highlighted with ESPript3.0.

The phrase "at a corresponding position" as used in the context of the present invention refers to the amino acid that aligns in an alignment of amino acid sequences with an amino acid sequence of a naturally occurring full length reference transposase, preferably with the full length amino acid sequence of SB according to SEQ ID NO: 1. The position of the reference transposase is determined from the first N-terminal amino acid. Accordingly, position 187 of SB within the full-length amino acid sequence according to SEQ ID NO: 1 is "H". Thus, an amino acid position corresponding to position 187 of SB in another transposase is an amino acid that aligns with "H" of SB at position 187 of SEQ ID NO: 1 in an alignment as exemplary shown in Figs. 1 and 2. Similarly, an amino acid position that corresponds to position 247 of SB is one that aligns with "P" of SB at position 247 of SEQ ID NO: 1, a position that corresponds to position 248 of SB is one that aligns with "K" of SB at position 248 of SEQ ID NO: 1.

In the context of present invention, the "primary structure" of a protein or polypeptide is the sequence of amino acids in the polypeptide chain. The "secondary structure" of a protein is the general three-dimensional form of local segments of the protein. It does not, however, describe specific atomic positions in three-dimensional space, which are considered to be tertiary structure. In proteins, the secondary structure is defined by patterns of hydrogen bonds between backbone amide and carboxyl groups. The "tertiary structure" of a protein is the three-dimensional structure of the protein determined by the atomic coordinates. The "quaternary structure" is the arrangement of multiple folded or coiled protein or polypeptide molecules in a multi-subunit complex.

The term "folding" or "protein folding" as used herein refers to the process by which a protein assumes its three-dimensional shape or conformation, i.e. whereby the protein is directed to form a specific three-dimensional shape through noncovalent and/or covalent interactions, such as but not limited to hydrogen bonding, metal coordination, hydrophobic forces, van der Waals forces, pi-pi interactions, electrostatic effects and/or intramolecular Cys bonds. The term "folded protein" thus, refers to the three-dimensional shape of parts or all of the protein, such as its secondary, tertiary, or quaternary structure.

The term "beta strand" as used in the context of the present invention refers to a 5 to 10 amino acid long section within a polypeptide chain in which the torsion angle of N-Cα-C-N in the backbone is about 120 degrees. Beta strands within a given protein sequence can be predicted after (multiple) sequence alignment (e.g. using Clustal omega) by retrieving annotations from pdb files. Prediction of beta strands can be performed using commonly available software tools like JPred. Start and end positions of the seven beta strands can be confirmed by additional structural alignment of PDB files using pyMol.

The terms "β sheet" or "beta sheet" as used interchangeably in the context of the present invention refers to two β-strands which form the β sheet. The β sheet is a common motif of regular secondary structure in proteins. Because peptide chains have a directionality conferred by their N-terminus and C-terminus, β-strands too can be said to be directional. They are usually represented in protein topology diagrams by an arrow pointing toward the C-terminus. Adjacent β-strands can form hydrogen bonds in antiparallel, parallel, or mixed arrangements. In an antiparallel arrangement, the successive β-strands alternate directions so that the N-terminus of one strand is adjacent to the C-terminus of the next. This is the arrangement that produces the strongest inter-strand stability because it allows the inter-strand hydrogen bonds between carbonyls and amines to be planar, which is their preferred orientation. A β structure is characterized by long extended polypeptide chains. The amino acid composition of β strands tends to favor hydrophobic (water fearing) amino acid residues. The side chains of these residues tend to be less soluble in water than those of more hydrophilic (water loving) residues. β structures tend to be found inside the core structure of proteins where the hydrogen bonds between strands are protected from competition with water molecules.

The terms "a-helix" or "alpha helix" as used interchangeably in the context of the present invention refers to a common motif in the secondary structure of proteins and is a right hand-helix conformation in which every backbone N-H group hydrogen bonds to the backbone C=O group of the amino acid located four residues earlier along the protein sequence. Among types of local structure in proteins, the α-helix is the most extreme and the most predictable from sequence, as well as the most prevalent.

The term "intervening region" or "loop" as used interchangeably in the context of the present invention refers to the amino acid residues between two β sheet sheets or a β sheet and α helix. Generally, the "intervening region" or "loop" are less structured or not structured at all, which provides them with flexibility.

To refer to a substitution within a given amino acid sequence the following designation is used "XNo.Z" means that the amino acid "X" of the original amino acid sequence at a particular position "No." is substituted by amino acid "Z", whereas "XNo.Y/X'No.'Z'/X"No."Z"" is intended to mean that original amino acids "X" at position "No.", "X" may be substituted alternatively with several different amino acids that are indicated as "Z", "Z" and "Z" respectively. The amino acid position is either indicated with respect to a specific length amino acid sequence, preferably a wild-type, full length sequence of a particular transposase.

The terms "vector" or "expression vector" are used interchangeably and refer to a polynucleotide or a mixture of a polynucleotide and proteins capable of being introduced or of introducing the collection of nucleic acids of the present invention or one nucleic acid that is part of the collection of nucleic acids of the invention into a cell, preferably a mammalian cell. Examples of vectors include but are not limited to plasmids, cosmids, phages, viruses or artificial chromosomes. In particular, a vector is used to transport the promoter and the collection of the nucleic acids or one nucleic acid that is part of the collection of nucleic acids of the invention into a suitable host cell. Expression vectors may contain "replicon" polynucleotide sequences that facilitate the autonomous replication of the expression vector in a host cell. Once in the host cell, the expression vector may replicate independently of or coincidental with the host chromosomal DNA, and several copies of the vector and its inserted DNA can be generated. In case that replication incompetent expression vectors are used - which is often the case for safety reasons - the vector may not replicate but merely direct expression of the nucleic acid. Depending on the type of expression vector the expression vector may be lost from the cell, i.e. only transiently expresses the neo-antigens encoded by the nucleic acid or may be stable in the cell. Expression vectors typically contain expression cassettes, i.e. the necessary elements that permit transcription of the nucleic acid into an mRNA molecule.

The term "viral vector" is used in the context of the present invention to refer to a single or double stranded nucleic acid sequence that can assemble into an infectious viral particle. This nucleic acid sequence may be a full or partial viral genome. In the latter case the viral genome preferably comprises one or more heterologous genes. For some viral particles only very short sequences of the viral genome are required to allow assembly of an infectious viral particle. For example, for assembly of an infectious adeno-associated viral particle only a short (about 200 bp long) repeat sequence placed at the 5' and 3' of a heterologous nucleic acid of a given length (typically between 4.5. to 5.3 kB for an adeno-associated virus) will allow assembly of infectious adeno-associated viral particles. The minimal nucleic acid sequence for assembly of a given virus are well known. The larger the viral genome and the smaller the minimal viral sequence for assembly of an infectious viral particle, the bigger the heterologous gene(s) can be that can be inserted into the viral vector.

"Sequence similarity" indicates the percentage of amino acids that either are identical or that represent conservative amino acid substitutions. The term "Sequence identity" between two amino acid sequences indicates the percentage of amino acids that are identical between two given sequences. The alignment for determining sequence similarity, preferably sequence identity, can be done with art known tools, preferably using the best sequence alignment, for example, using CLC main Workbench (CLC bio) or Align, using standard settings, preferably EMBOSS::needle, Matrix: Blosum62, Gap Open 10.0, Gap Extend 0.5. The percentage of identity is determined with reference to the full-length sequence that is used for comparison and not just for the sequence or sequence stretch with the highest similarity. Thus, an amino acid that shares 100% sequence identity to 50 consecutive amino acids of the 100 amino acid long sequence that is used for comparison only has 50% sequence identity (on the assumption that there are no further amino acids that share any identity outside the 50 consecutive amino acids.

The term "pharmaceutically acceptable", as used herein, refers to the non-toxicity of a material, which, preferably, does not interact with the action of the active agent of the pharmaceutical composition. In particular, "pharmaceutically acceptable" means approved by a regulatory agency of the Federal or a state government or listed in the U.S. Pharmacopoeia, European Pharmacopoeia or other generally recognized pharmacopeia for use in animals, and more particularly in humans.

The term "carrier" refers to an organic or inorganic component, of a natural or synthetic nature, in which the active component is combined in order to facilitate, enhance or enable application. According to the invention, the term "carrier" also includes one or more compatible solid or liquid fillers, diluents or encapsulating substances, which are suitable for administration to a subject. Possible carrier substances (e.g., diluents) are, for example, sterile water, Ringer's solution, Lactated Ringer's solution, physiological saline, bacteriostatic saline (e.g., saline containing 0.9% benzyl alcohol), phosphate-buffered saline (PBS), Hank's solution, fixed oils, polyalkylene glycols, hydrogenated naphthalenes and biocompatible lactide polymers, lactide/glycolide copolymers or polyoxyethylene/polyoxy-propylene copolymers. In one embodiment, the carrier is PBS. The resulting solutions or suspensions are preferably isotonic to the blood of the recipient. Suitable carriers and their formulations are described in greater detail in Remington's Pharmaceutical Sciences, 17th ed., 1985, Mack Publishing Co.

The term "cell" is used in the context of the present invention to refer to a eukaryotic or a prokaryotic cell, such as a bacterial cell, a yeast cell, or a cell of a mammal, preferably a cell of a human, a mouse, a rat, a rabbit, a dog, a monkey, or a cat.

### Aspects of the invention and preferred embodiments

In the following different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In the work leading to the present invention, it was surprisingly shown that variants of a polypeptide with transposase activity have a remarkable gain of specificity of integration into the genome.

In a first aspect, the present invention provides a polypeptide with transpositional activity comprising, consisting essentially of, or consisting of a variant of a naturally occurring transposase with the following secondary structural elements:
α1 helix- α2 helix- β1 sheet- β2 sheet- β3 sheet-β4 sheet- β5 sheet-α3 helix-β6 sheet- η1-α4 helix-η2-α5 helix- α6 helix-α7 helix-α8 helix
wherein
   (i) a naturally occurring H in the loop connecting the β3 sheet and the β4 sheet is substituted with V, A, N, C, Q, G, I, L, M, F, P, R, S, T, W, K or Y, preferably with V or P, or
      a naturally occurring F in the loop connecting the β3 sheet and the β4 sheet is substituted with V, A, N, C, Q, G, I, L, M, P, R, S, T, W, K, H or Y, preferably with V or P, or
      a naturally occurring Y in the loop connecting the β3 sheet and the β4 sheet is substituted with V, A, N, C, Q, G, I, L, M, F, P, R, S, T, W, H or K, preferably with V or P, or
      a naturally occurring L in the loop connecting the β3 sheet and the β4 sheet is substituted with V, A, N, C, Q, G, I, M, F, P, R, S, T, W, H, Y or K, preferably with V or P, or
      a naturally occurring S in the loop connecting the β3 sheet and the β4 sheet is substituted with V, A, N, C, Q, G, I, L, M, F, P, R, T, W, H, Y or K, preferably with V or P;
         and/or
   (ii) at least one naturally occurring Q, P, S, or T in η1 connecting the β6 sheet with α4 helix is substituted with R, S or A, preferably with R;
      and/or
   (iii) at least one naturally occurring K, I, S, T, V, P or C in η1 connecting the β6 sheet with α4 helix is preferably substituted with R;
   wherein the variant of the naturally occurring transposase comprises at least one of the substations (i), (ii) or (iii).

The present invention is based on the surprising discovery that substituting certain amino acids in transposases leads to transposases which gain specificity of integration into the genome, in particular into palindromic AT repeat target sequence, that enhance the DNA bendability of target sites enriched in non-nucleosomal DNA and de-target exons as well as transcriptional regulatory regions of genes in the human genome. All these properties contribute to the enhancing of the safety and utility of the polypeptides of the invention in gene therapy applications. These properties are very favourable for a transposase because it is less likely to inactive or mutate an expressed gene. The improved integration pattern can be assessed by the experiments described in Example 3 of the present application and presented in Fig. 5. It is, thus preferred that the number of integration events into exons is reduced in the polypeptide of the present invention by at least 25%, more preferably at least 40% or more preferably at least 50%, if compared to the number of integration events into exons observed when using SB according to SEQ ID NO: 1, if the polypeptide comprises or consists of a variant of SB according to SEQ ID NO: 1.

Preferably the variant of the invention retains the transpositional activity of the full length wild-type transposase, i.e. has at least 100% of the transpositional activity of the full length wild type transposase on which the variant is based, preferably 100% of the transpositional activity of SB according to SEQ ID NO: 1.

Fig. 1 and Fig. 2 depict alignments of the amino acid sequences of transposases of different degrees of relatedness to SB. While the amino acid sequence difference is substantial at least between some of the transposases included in the alignment of Fig. 1 and SB, all transposases share a similar secondary structure. They comprise elements of secondary structure like α helixes and β sheets in a certain order and of a certain length. Figs. 1 and 2 indicate the positions of α1 helix-α2 helix-β1 sheet- β2 sheet-β3 sheet-β4 sheet-β5 sheet-α3 helix-β6 sheet-η1-α4 helix-η2-α5 helix- α6 helix-α7 helix-α8 helix within these amino acid sequences. With respect to SB the elements α1 helix-α2 helix-β1 sheet-β2 sheet-β3 sheet-β4 sheet-β5 sheet-α3 helix-β6 sheet-η1-α4 helix-η2-α5 helix-α6 helix-α7 helix-α8 helix span the following amino acids of SEQ ID NO: 1:
α1 helix: AA 128 to 138 of SEQ ID NO: 1;
α2 helix: AA 143 to 147 of SEQ ID NO: 1;
β1 sheet: AA 149 to 158 of SEQ ID NO: 1;
β2 sheet: AA 169 to 171 of SEQ ID NO: 1;
β3 sheet: AA 173 to 176 of SEQ ID NO: 1;
β4 sheet: AA 191 to 199 of SEQ ID NO: 1;
β5 sheet: AA 202 to 208 of SEQ ID NO: 1;
α3 helix: AA 215 to 233 of SEQ ID NO: 1;
β6 sheet: AA 240 to 241 of SEQ ID NO: 1;
η1: AA 247 to 250 of SEQ ID NO: 1;
α4 helix: AA 252 to 260 of SEQ ID NO: 1;
η2: AA 273 to 275 of SEQ ID NO: 1;
α5 helix: AA 278 to 291 of SEQ ID NO: 1;
α6 helix: AA 297 to 309 of SEQ ID NO: 1;
α7 helix: AA 313 to 321 of SEQ ID NO: 1;
α8 helix: AA 323 to 332 of SEQ ID NO: 1;

Thus, α1 helix-α2 helix-β1 sheet-β2 sheet-β3 sheet-β4 sheet-β5 sheet-α3 helix-β6 sheet-η1-α4 helix-η2-α5 helix-α6 helix-α7 helix-α8 helix of other transposases will begin and end at amino acid positions corresponding to the above indicated amino acid positions within SB. This allows the skilled person to determine the secondary structural elements: α1 helix-α2 helix-β1 sheet-β2 sheet-β3 sheet-β4 sheet-β5 sheet-α3 helix-β6 sheet-η1-α4 helix-η2-α5 helix-α6 helix-α7 helix-α8 helix within any given transposase and to correspondingly determine the loop connecting the β3 sheet and the β4 sheet and the amino acid in η1 connecting the β6 sheet with α4 helix within any given transposase.

Furthermore, the skilled person can analyse the three-dimensional structure and amino acid sequence in relation to that structure in other transposases and predict the alignment of amino acids based on the three-dimensional structures. Moreover, computer programs can assist in predicting the secondary structure of a protein or polypeptide of interest, i.e. for example of any other transposase of interest. One method is based on homology modelling. It is usual that, two polypeptides or proteins which have a sequence identity of greater than 30%, or similarity greater than 40% often have similar structural topologies. The growth of the protein structural database (PDB) has provided enhanced predictability of secondary structure, including the potential number of folds within a polypeptide's or protein's structure. Further methods of predicting secondary structure of proteins and polypeptides are known in the art and include for example threading, profile analysis, and evolutionary linkage. Thus, a skilled person can easily add further transposases of this superfamily to the alignment allowing the identification of amino acids corresponding to amino acid position 187, 247 and 248 of SB in these transposases, and/or to the amino acids in the loop connecting the β3 sheet and the β4 sheet of any given transposase, or the amino acid in η1 connecting the β6 sheet with α4 helix of any given transposase.

The loop connecting the β3 sheet and the β4 sheet of a given transposase may comprise in the naturally occurring sequence an H, F, L, S or Y. In this case it is preferred to substitute these amino acids with another amino acid that is likely to alter the protein-DNA interaction of the transposase.

If the naturally occurring amino acid in the loop connecting the β3 sheet and the β4 sheet is H it is preferred that this amino acid is substituted with V, A, N, C, Q, G, I, L, M, F, P, R, S, T, W, K or Y, preferably with V, I, L, M, S, or T, more preferably with V, I or L, most preferably with V or P.

If the naturally occurring amino acid in the loop connecting the β3 sheet and the β4 sheet is F it is preferred that this amino acid is substituted with V, A, N, C, Q, G, I, L, M, P, R, S, T, W, K or Y, preferably with V, I, L, M, S, or T, more preferably with V, I or L, most preferably with V or P.

If the naturally occurring amino acid in the loop connecting the β3 sheet and the β4 sheet is Y it is preferred that this amino acid is substituted with V, A, N, C, Q, G, I, L, M, F, P, R, S, T, W, or K, preferably with V, I, L, M, S, or T, more preferably with V, I or L, most preferably with V or P.

If the naturally occurring amino acid in the loop connecting the β3 sheet and the β4 sheet is L it is preferred that this amino acid is substituted with V, A, N, C, Q, G, I, M, F, P, R, S, T, W, H, Y or K, preferably with V or P.

If the naturally occurring amino acid in the loop connecting the β3 sheet and the β4 sheet is S it is preferred that this amino acid is substituted with V, A, N, C, Q, G, I, L, M, F, P, R, T, W, H, Y or K, preferably with V or P.

The η1 connecting the β6 sheet with α4 helix of a given transposase may comprise in the naturally occurring sequence K, I, S, T, V, P or C (position 248 in SB) or Q, P, S, or T (position 247 in SB). In this case it is preferred to substitute these amino acids with another amino acid that is likely to alter the protein DNA interaction of the transposase.

If the naturally occurring amino acid in η1 connecting the β6 sheet with α4 helix is at least one naturally occurring Q, P, S, or T it is preferred that this amino acid is substituted with R or A, preferably with R.

If the naturally occurring amino acid in η1 connecting the β6 sheet with α4 helix is at least one naturally occurring K, I, S, T, V, P or C it is preferred that this amino acid is substituted with R, A, P, or Q, preferably with R.

In a further preferred embodiment both a naturally occurring Q, P, S, or T and a K, I, S, T, V, P or C in η1 connecting the β6 sheet with α4 helix is substituted as outlined above, preferably both are substituted with R.

In a further embodiment or in another aspect of the present invention the substitution may also occur in a variant of a naturally occurring transposase. The term "variant of a naturally occurring transposase" refers to an amino acid sequence that has at least 70% amino acid sequence identity to the amino acid sequence of a transposase of a naturally occurring transposase, preferably to one of the transposases according to SEQ ID NO: 1 to 17. More preferably a variant of a transposase that is further modified by one or more substitutions as outlined above has at least 75%, 80%, 85%, 90%, 95%, 96%, 97%, 98% or 99% sequence identity to the amino acid sequence of the transposase of a naturally occurring transposase, preferably to one of the transposases according to SEQ ID NO: 1 to 17, most preferably to the SB transposase according to SEQ ID NO: 1. If the substitutions of the invention are in the context of a variant or a naturally occurring transposase, the degree of identity is determined without the substitution of the invention. Thus, a variant of a naturally occurring transposase may have 70 amino acid sequence identity to SEQ ID NO: 1 and additionally a substitution according to (i), (ii) and/or (iii) as outlined above.

It is further preferred that the number of integration events into exons is reduced in the polypeptide of the present invention based on the amino acid sequence of SEQ ID NO: 1 to 17 by at least 25%, more preferably at least 40% or more preferably at least 50%, if compared to the number of integration events into exons observed when using a transposase according to SEQ ID NO: 1 to 17, respectively, if the polypeptide of the invention comprises or consists of a variant of a transposase according to SEQ ID NO: 1 to 17.

In a preferred embodiment the polypeptide of the present invention comprises, consists or essentially consists of the full length naturally occurring transposase, preferable comprises consists or essentially consists of the full length naturally occurring transposase according to SEQ ID NO: 1 to 17 or a variant thereof. In this preferred embodiment the reference amino acid sequence for the determination of the variant is the full-length sequence. Thus, in this preferred embodiment that polypeptide of the present invention the variant comprises, consists or essentially consists of an amino acid sequence that has at least 70%, 75%, 80%, 85%, 90%, 95%, 96%, 97%, 98% or 99% sequence identity to the amino acid sequence of a naturally occurring transposase, preferably to a transposase according to SEQ ID NO: 1 to 17, most preferably to the transposase SB according to SEQ ID NO: 1. Again the amino acid sequence identity of the variant of a naturally occurring transposase is determined in the absence of the substitutions of the invention according to (i), (ii) and/or (iii).

In each of the above cases a variant of the invention retains transpositional activity of the naturally occurring or wild-type transposase. The amino acid changes may have an effect on the producibility and/or stability of the resulting transposases or may affect another activity, e.g. the transpositional activity. Substitutions that increase the transpositional activity of transposases, in particular of SB are described further below and have previously been described in WO 2009/003671. The presence of such substitutions in addition to the substitutions according to (i), (ii) and/or (iii) described above, are particularly preferred.

In one embodiment of the first aspect of the present invention, the naturally occurring transposase is selected from the group consisting of Sleeping Beauty (SB) (SEQ ID NO: 1), Tdrl (SEQ ID NO: 2), ZB (SEQ ID NO: 3), FP (SEQ ID NO: 4), Passport (SEQ ID NO: 5), TCB2 (SEQ ID NO: 6), S (SEQ ID NO: 7), Quetzal (SEQ ID NO: 8), Paris (SEQ ID NO: 9), Tc1 (SEQ ID NO: 10), Minos (SEQ ID NO: 11), Uhu (SEQ ID NO: 12), Bari (SEQ ID NO: 13), Tc3 (SEQ ID NO: 14), Impala (SEQ ID NO: 15), Himar (SEQ ID NO: 16), or Mos1 (SEQ ID NO: 17), preferably the transposase is SB (SEQ ID NO: 1), ZB (SEQ ID NO: 3), FP (SEQ ID NO: 4), Passport (SEQ ID NO: 5), or Minos (SEQ ID NO: 11). In the most preferred embodiment the transposase is SB (SEQ ID NO: 1).

In a particular preferred embodiment or in a further aspect of the invention, the polypeptide of the present invention comprises, consists or essentially consists of the transposase of SEQ ID NO: 1 to 17 or a variant thereof comprising at least one of the substations according to (i), (ii) or (iii) outlined above.

The transposases that are closely related to SB not only share secondary structural elements that are arranged in the above indicated order but also share significant amino acid similarity or even identity in certain key regions involved in DNA-protein interaction. Thus, the substitutions of the inventions in this subgroup of transposases that are closely related to SB can be characterized by consensus amino acid sequences that surround the amino acid at position 187 of SB. This consensus sequence is X₁X₂X₃Y₁X₄X₅GX₆ (SEQ ID NO: 18) with the meaning for the variables indicated in the following. The other consensus amino acid sequence that surrounds the amino acid sequences at position 247 and 248 of SB is X₇X₈DX₉X₁₀Y₂X₁₁Y₃H (SEQ ID NO: 19), wherein the variables have the meanings indicated in the following. A given transposase that is closely related to SB may comprise an amino acid sequence that fulfills the consensus sequence according to SEQ ID NO: 18 or SEQ ID NO: 19 or both. It is preferred that the transposase that is closely related to SB comprises amino acid sequences fulfilling both consensus sequences. Such transposes are highly similar to SB both in a region surrounding amino acid position 187 of SB and surrounding amino acid positions 247 and 248. Thus, the substitutions (i), (ii), and/or (iii) of the present invention are preferably comprised in a transposase comprising two consecutive amino acid sequence that fulfill the consensus according to SEQ ID NO: 18 and SEQ ID NO: 19, respectively. Accordingly, in one embodiment, the polypeptide of the first aspect of the present invention or alternatively in another aspect the present invention relates to a polypeptide with transpositional activity comprising, consisting essentially of, or consisting of a variant of a naturally occurring transposase, which comprises
(i) a first amino acid stretch:
   X₁X₂X₃Y₁X₄X₅GX₆ (SEQ ID NO: 18), wherein
   X₁ is T, F, P, or R,
   X₂ is I, T, N, R, K, Q, or V,
   X₃ is K, N, L, R or Q, preferably K,
   X₄ is G, N, P, A, or Q, preferably G,
   X₅ is G, K, A, or is absent, preferably X₅ is G,
   X₆ is S, or is absent, and
   Y₁ is F, H, Y, L or S in the wild-type protein and
   wherein when Y₁ is F in the naturally occurring transposase and is unsubstituted or is substituted with A, N, C, Q, G, I, L, M, P, R, S, T, V, W, K or Y, preferably with V or P;
   wherein when Y₁ is H in the naturally occurring transposase and is unsubstituted or is substituted with A, N, C, Q, G, I, L, M, F, P, R, S, T, V, W, K or Y, preferably with V or P;
   wherein when Y₁ is Y in the naturally occurring transposase and is unsubstituted or is substituted with A, N, C, Q, G, I, L, M, F, P, R, S, T, V, W, or K, preferably with V or P;
   wherein when Y₁ is L in the naturally occurring transposase and is unsubstituted or is substituted with A, N, C, Q, G, I, F, M, P, R, S, T, V, W, K or Y, preferably with V or P;
   wherein when Y₁ is S in the naturally occurring transposase and is unsubstituted or is substituted with A, N, C, Q, G, I, L, F, M, P, R, T, V, W, K or Y, preferably with V or P;
   and/or
(ii) a second amino acid stretch:
   X₇X₈DX₉X₁₀Y₂X₁₁Y₃H (SEQ ID NO: 19), wherein
   X₇ is Q, L, M, H or Y, and
   X₈ is M, Q or H,
   X₉ is N, H or G
   X₁₀ is A, or D,
   X₁₁ is V, T or K, and
   Y₂ is Q, P, S, or T in the naturally occurring transposase and is unsubstituted or is substituted with R or A, preferably with R,
      and/or
   Y₃ is K, I, S, T, V, P or C in the naturally occurring transposase and is unsubstituted or is substituted with R, A, P, or Q, preferably with R,
   wherein at least one of Y₁, Y₂ and Y₃ is substituted.

In a preferred embodiment,
X₁ is T,
X₂ is V or I, preferably V,
X₃ is K or Q, preferably K,
X₄ is G,
X₅ is G,
X₆ is S, and
Y₁ is F, H, Y, L or S in the wild-type protein and
wherein when Y₁ is F in the naturally occurring transposase and is unsubstituted or is substituted with A, N, C, Q, G, I, L, M, P, R, S, T, V, W, K or Y, preferably with V or P;
wherein when Y₁ is H in the naturally occurring transposase and is unsubstituted or is substituted with A, N, C, Q, G, I, L, M, F, P, R, S, T, V, W, K or Y, preferably with V or P;
wherein when Y₁ is Y in the naturally occurring transposase and is unsubstituted or is substituted with A, N, C, Q, G, I, L, M, F, P, R, S, T, V, W, or K, preferably with V or P;
wherein when Y₁ is L in the naturally occurring transposase and is unsubstituted or is substituted with A, N, C, Q, G, I, F, M, P, R, S, T, V, W, K or Y, preferably with V or P;
wherein when Y₁ is S in the naturally occurring transposase and is unsubstituted or is substituted
with A, N, C, Q, G, I, L, F, M, P, R, T, V, W, K or Y, preferably with V or P;
   and/or
X₇ is Q,
X₈ is M, Q or H,
X₉ is N, H, or G
X₁₀ is A, or D,
X₁₁ is T, V or K
Y₂ is P in the naturally occurring transposase and is unsubstituted or has been substituted with R or A, preferably with R, and/or
Y₃ is K in the naturally occurring transposase and is unsubstituted or has been substituted with R,
A, P, or Q, preferably with R, and
wherein at least one of Y₁, Y₂ and Y₃ is substituted.

If any of these substitutions are present in a variant of a transposase the sequence identity of the variant to the respective wild-type transposase sequence is again determined prior to the introduction of a substitution according to (i), (ii), and/or (iii).

In one embodiment, the polypeptide according to the invention, comprises SEQ ID NO: 1 or variants thereof with transpositional activity and at least 70%, 80%, 85%, 90%, 95%, 96%, 97%, 98%, 99% or 100% sequence identity to SEQ ID NO: 1, and wherein the SEQ ID NO: 1 or the variant thereof comprises one or more of the following substitutions:
(i) K248R, H187A, H187N, H187C, H187Q, H187G, H187I, H187L, H187M, H187F, H187S, H187V, H187W, or H187Y, preferably H187I, H187V or H187L, more preferably H187V; and/or
(ii) P247R; and/or
(iii) K248A, or K248S; preferably K248R;
or a substitution at a corresponding position of the variant thereof.

As noted above, various variants of naturally occurring transposases have been described. For example, WO 2009/003671 describes variants of transposases in particular of SB that have are hyperactive, i.e. that have an increased transpositional activity in comparison to the wild-type transposase, in particular SB. It is preferred that the substitutions of the invention are introduced into variants of the transposase that comprise substitutions or groups of substitutions that enhance one or more property, in particular the transpositional activity of the transposase. Thus, in one embodiment, the polypeptide according to the invention further comprises at least one of the following substitutions or groups of substitutions:
(1) K14R, K13D, K13A, K30R, K33A, T83A, I100L, R115H, R143L, R147E, A205K/H207V/K208R/D210E, H207V/K208R/D210E, R214D/K215A/E216V/N217Q; M243Q, E267D, T314N, and/or G317E;
(2) K14R//R214D/K215A/E216V/N217Q;
(3) K33A/R115H/R214D/K215A/E216V/N217Q/M243H;
(4) K14R/K30R/A205K/H207V/K208R/D210E/R214D/K215A/E216V/N217Q/M243H;
(5) K13D/K33A/T83N/H207V/K208R/D210E/M243Q;
(6) K13A/K33A/R214D/K215A/E216V/N217Q;
(7) K33A/T83N/R214D/K215NE216V/N217QHG317E;
(8) K14R/T83A/M243Q;
(9) K14R/T83A/I100L/M243Q;
(10) K14R/T83A/R143L/M243Q;
(11) K14R/T83A/R147E/M243Q;
(12) K14R/T83A/M243Q/E267D;
(13) K14R/T83A/M243Q/T314N;
(14) K14R/K30R/I110L/A205K/H207V/K208R/D210E/R214D/K215A/ E216V/N217Q/M243H;
(15) K14R/K30R/R143L/A205K/H207V/K208R/D210E/R214D/K215A/ E216V/N217Q//M243H;
(16) K14R/K30R/R147E/A205K/H207V/K208R/D210E/R214D/K215A/ E216V/N217Q/M243H;
(17) K14R/K30R/A205K/H207V/K208R/D210E/R214D/K215A/ E216V/N217Q//M243H/E267D;
(18) K14R/K30R/A205K/H207V/K208R/D210E/R214D/K215A/ E216V/N217Q/M243H/T314N;
(19) K14R/K30R/A205K/H207V/K208R/D210E/R214D/K215A/ E216V/N217Q//M243H/G317E;
(20) K14R/K33A/R115H/R214D/K215A/E216V/N217Q/M243H;
(21) K14R/K30R/R147E/A205K/H207V/K208R/D210E/R214D/K215A/ E216V/N217Q//M243H/T314N;
(22) K14R/K30R/R143U/A205K/H207V/K208R/D210E/R214D/K215A/ E216V/N217Q//M243H/E267D;
(23) K14R/K30R/R143L/A205K/H207V/K208R/D210E/R214D/K215A/ E216V/N217Q//M243H/T314N;
(24) K14R/K30R/R143L/A205K/H207V/K208R/D210E/R214D/K215A/ E216V/N217Q//M243H/G317E;
(25) K14R/K33A/R115H/R143L/R214D/K215A/E216V/N217Q/M243H;
(26) K14R/K33A/R115H/R147E//R214D/K215A/E216V/N217Q/M243H;
(27) K14R/K33A/R115H/R214D/K215A/E216V/N217Q//M243H/E267D;
(28) K14R/K33A/R115H/R214D/K215A/E216V/N217Q//M243H/T314N;
(29) K14R/K33A/R115H/R214D/K215A/E216V/N217Q//M243H/G317E;
(30) K14R/T83A/M243Q/G317E; or
(31) K13A/K33A/T83N/R214D/K215A/E216V/N217Q;
or at least one of the following substitution(s) or groups of substitutions at corresponding position(s) of the variant thereof. Preferably, the above outlined substitution(s) or groups of substitutions are introduced at corresponding positions into the naturally occurring transposase selected from the group consisting of Tdr1 (SEQ ID NO: 2), ZB (SEQ ID NO: 3), FP (SEQ ID NO: 4), Passport (SEQ ID NO: 5), TCB2 (SEQ ID NO: 6), S (SEQ ID NO: 7), Quetzal (SEQ ID NO: 8), Paris (SEQ ID NO: 9), Tc1 (SEQ ID NO: 10), Minos (SEQ ID NO: 11), Uhu (SEQ ID NO: 12), Bari (SEQ ID NO: 13), Tc3 (SEQ ID NO: 14), Impala (SEQ ID NO: 15), Himar (SEQ ID NO: 16), or Mos1 (SEQ ID NO: 17), preferably the transposase is ZB (SEQ ID NO: 3), FP (SEQ ID NO: 4), Passport (SEQ ID NO: 5), or Minos (SEQ ID NO: 11) in addition to the substitutions of the invention, preferably (i) K248R, H187A, H187N, H187C, H187Q, H187G, H187I, H187L, H187M, H187F, H187S, H187V, H187W, or H187Y, preferably H187I, H187V or H187L, more preferably H187V; and/or (ii)P247R; and/or (iii)K248A, or K248S; preferably K248R; or a substitution at a corresponding position of Tdrl (SEQ ID NO: 2), ZB (SEQ ID NO: 3), FP (SEQ ID NO: 4), Passport (SEQ ID NO: 5), TCB2 (SEQ ID NO: 6), S (SEQ ID NO: 7), Quetzal (SEQ ID NO: 8), Paris (SEQ ID NO: 9), Tc1 (SEQ ID NO: 10), Minos (SEQ ID NO: 11), Uhu (SEQ ID NO: 12), Bari (SEQ ID NO: 13), Tc3 (SEQ ID NO: 14), Impala (SEQ ID NO: 15), Himar (SEQ ID NO: 16), or Mos1 (SEQ ID NO: 17), preferably the transposase is SB (SEQ ID NO: 1), ZB (SEQ ID NO: 3), FP (SEQ ID NO: 4), Passport (SEQ ID NO: 5), or Minos (SEQ ID NO: 11).

The polypeptide of the first aspect of the invention most preferably comprises or consists of an amino acid sequence that is based on SEQ ID NO: 1 and wherein the following amino acids are substituted within the amino acid sequence of SEQ ID NO: 1:(1) K14R increasing the transpositional activity and H187I, H187V or H187L, more preferably H187V, and/or P247R; and/or K248A, or K248S; preferably K248R;
(2) K13D increasing the transpositional activity and H187I, H187V or H187L, more preferably H187V, and/or P247R; and/or K248A, or K248S; preferably K248R;
(3) K13A increasing the transpositional activity and H187I, H187V or H187L, more preferably H187V, and/or P247R; and/or K248A, or K248S; preferably K248R;
(4) K30R increasing the transpositional activity and H187I, H187V or H187L, more preferably H187V, and/or P247R; and/or K248A, or K248S; preferably K248R;
(5) K33A increasing the transpositional activity and H187I, H187V or H187L, more preferably H187V, and/or P247R; and/or K248A, or K248S; preferably K248R;
(6) T83A increasing the transpositional activity and H187I, H187V or H187L, more preferably H187V, and/or P247R; and/or K248A, or K248S; preferably K248R;
(7) I100L increasing the transpositional activity and H187I, H187V or H187L, more preferably H187V, and/or P247R; and/or K248A, or K248S; preferably K248R;
(8) R115H increasing the transpositional activity and H187I, H187V or H187L, more preferably H187V, and/or P247R; and/or K248A, or K248S; preferably K248R;
(9) R143L increasing the transpositional activity and H187I, H187V or H187L, more preferably H187V, and/or P247R; and/or K248A, or K248S; preferably K248R;
(10) R147E increasing the transpositional activity and H187I, H187V or H187L, more preferably H187V, and/or P247R; and/or K248A, or K248S; preferably K248R;
(11) A205K/H207V/K208R/D210E increasing the transpositional activity and H187I, H187V or H187L, more preferably H187V, and/or P247R; and/or K248A, or K248S; preferably K248R;
(12) H207V/K208R/D210E increasing the transpositional activity and H187I, H187V or H187L, more preferably H187V, and/or P247R; and/or K248A, or K248S; preferably K248R;
(13) R214D/K215A/E216V/N217Q increasing the transpositional activity and H187I, H187V or H187L, more preferably H187V, and/or P247R; and/or K248A, or K248S; preferably K248R;
(14) M243Q increasing the transpositional activity and H187I, H187V or H187L, more preferably H187V, and/or P247R; and/or K248A, or K248S; preferably K248R;
(15) E267D increasing the transpositional activity and H187I, H187V or H187L, more preferably H187V, and/or P247R; and/or K248A, or K248S; preferably K248R;
(16) T314N increasing the transpositional activity and H187I, H187V or H187L, more preferably H187V, and/or P247R; and/or K248A, or K248S; preferably K248R;
(17) G317E increasing the transpositional activity and H187I, H187V or H187L, more preferably H187V, and/or P247R; and/or K248A, or K248S; preferably K248R;
(18) Combinations of two or more of the single substitutions or groups of substitutions increasing the transpositional activity indicated under (1) to (18), are combined with substitutions H187I, H187V or H187L, more preferably H187V, and/or P247R; and/or K248A, or K248S; preferably K248R;
   Preferred combinations of the substitutions increasing the transpositional activity indicated under (1) to (18) with the substitutions of the inventions are the following:
   (19) K14R//R214D/K215A/E216V/N217Q increasing the transpositional activity and H187I, H187V or H187L, more preferably H187V, and/or P247R; and/or K248A, or K248S; preferably K248R;
   (20) K33A/R115H/R214D/K215A/E216V/N217Q/M243H increasing the transpositional activity and H187I, H187V or H187L, more preferably H187V, and/or P247R; and/or K248A, or K248S; preferably K248R;
   (21) K14R/K30R/A205K/H207V/K208R/D210E/R214D/K215A/E216V/N217Q/M243H increasing the transpositional activity and H187I, H187V or H187L, more preferably H187V, and/or P247R; and/or K248A, or K248S; preferably K248R increasing the transpositional activity and H187I, H187V or H187L, more preferably H187V, and/or P247R; and/or K248A, or K248S; preferably K248R;
   (22) K13D/K33A/T83N/H207V/K208R/D210E/M243Q increasing the transpositional activity and H187I, H187V or H187L, more preferably H187V, and/or P247R; and/or K248A, or K248S; preferably K248R;
   (23) K13A/K33A/R214D/K215A/E216V/N217Q increasing the transpositional activity and H187I, H187V or H187L, more preferably H187V, and/or P247R; and/or K248A, or K248S; preferably K248R;
   (24) K33A/T83N/R214D/K215NE216V/N217Q//G317E increasing the transpositional activity and H187I, H187V or H187L, more preferably H187V, and/or P247R; and/or K248A, or K248S; preferably K248R;
   (25) K14R/T83A/M243Q increasing the transpositional activity and H187I, H187V or H187L, more preferably H187V, and/or P247R; and/or K248A, or K248S; preferably K248R;
   (26) K14R/T83A/I100L/M243Q increasing the transpositional activity and H187I, H187V or H187L, more preferably H187V, and/or P247R; and/or K248A, or K248S; preferably K248R;
   (27) K14R/T83A/R143L/M243Q increasing the transpositional activity and H187I, H187V or H187L, more preferably H187V, and/or P247R; and/or K248A, or K248S; preferably K248R;
   (28) K14R/T83A/R147E/M243Q increasing the transpositional activity and H187I, H187V or H187L, more preferably H187V, and/or P247R; and/or K248A, or K248S; preferably K248R;
   (29) K14R/T83A/M243Q/E267D increasing the transpositional activity and H187I, H187V or H187L, more preferably H187V, and/or P247R; and/or K248A, or K248S; preferably K248R;
   (30) K14R/T83A/M243Q/T314N increasing the transpositional activity and H187I, H187V or H187L, more preferably H187V, and/or P247R; and/or K248A, or K248S; preferably K248R;
   (31) K14R/K30R/I110L/A205K/H207V/K208R/D210E/R214D/K215A/ E216V/N217Q/M243H increasing the transpositional activity and H187I, H187V or H187L, more preferably H187V, and/or P247R; and/or K248A, or K248S; preferably K248R;
   (32) K14R/K30R/R143L/A205K/H207V/K208R/D210E/R214D/K215A/ E216V/N217Q//M243H increasing the transpositional activity and H187I, H187V or H187L, more preferably H187V, and/or P247R; and/or K248A, or K248S; preferably K248R;
   (33) K14R/K30R/R147E/A205K/H207V/K208R/D210E/R214D/K215A/ E216V/N217Q/M243H increasing the transpositional activity and H187I, H187V or H187L, more preferably H187V, and/or P247R; and/or K248A, or K248S; preferably K248R;
   (34) K14R/K30R/A205K/H207V/K208R/D210E/R214D/K215A/ E216V/N217Q//M243H/E267D increasing the transpositional activity and H187I, H187V or H187L, more preferably H187V, and/or P247R; and/or K248A, or K248S; preferably K248R;
   (35) K14R/K30R/A205K/H207V/K208R/D210E/R214D/K215A/ E216V/N217Q/M243H/T314N increasing the transpositional activity and H187I, H187V or H187L, more preferably H187V, and/or P247R; and/or K248A, or K248S; preferably K248R;
   (36) K14R/K30R/A205K/H207V/K208R/D210E/R214D/K215A/ E216V/N217Q//M243H/G317E increasing the transpositional activity and H187I, H187V or H187L, more preferably H187V, and/or P247R; and/or K248A, or K248S; preferably K248R;
   (37) K14R/K33A/R115H/R214D/K215A/E216V/N217Q/M243H; increasing the transpositional activity and H187I, H187V or H187L, more preferably H187V, and/or P247R; and/or K248A, or K248S; preferably K248R; (38)
      K14R/K30R/R147E/A205K/H207V/K208R/D210E/R214D/K215A/ E216V/N217Q//M243H/T314N;
   (39) K14R/K30R/R143U/A205K/H207V/K208R/D210E/R214D/K215A/ E216V/N217Q//M243H/E267D increasing the transpositional activity and H187I, H187V or H187L, more preferably H187V, and/or P247R; and/or K248A, or K248S; preferably K248R;
   (40) K14R/K30R/R143L/A205K/H207V/K208R/D210E/R214D/K215A/ E216V/N217Q//M243H/T314N increasing the transpositional activity and H187I, H187V or H187L, more preferably H187V, and/or P247R; and/or K248A, or K248S; preferably K248R;
   (41) K14R/K30R/R143L/A205K/H207V/K208R/D210E/R214D/K215A/ E216V/N217Q//M243H/G317E increasing the transpositional activity and H187I, H187V or H187L, more preferably H187V, and/or P247R; and/or K248A, or K248S; preferably K248R;
   (42) K14R/K33A/R115H/R143L/R214D/K215A/E216V/N217Q/M243H increasing the transpositional activity and H187I, H187V or H187L, more preferably H187V, and/or P247R; and/or K248A, or K248S; preferably K248R;
   (43) K14R/K33A/R115H/R147E//R214D/K215A/E216V/N217Q/M243H increasing the transpositional activity and H187I, H187V or H187L, more preferably H187V, and/or P247R; and/or K248A, or K248S; preferably K248R;
   (44) K14R/K33A/R115H/R214D/K215A/E216V/N217Q//M243H/E267D increasing the transpositional activity and H187I, H187V or H187L, more preferably H187V, and/or P247R; and/or K248A, or K248S; preferably K248R;
   (45) K14R/K33A/R115H/R214D/K215A/E216V/N217Q//M243H/T314N increasing the transpositional activity and H187I, H187V or H187L, more preferably H187V, and/or P247R; and/or K248A, or K248S; preferably K248R;
   (46) K14R/K33A/R115H/R214D/K215A/E216V/N217Q//M243H/G317E increasing the transpositional activity and H187I, H187V or H187L, more preferably H187V, and/or P247R; and/or K248A, or K248S; preferably K248R;
   (47) K14R/T83A/M243Q/G317E increasing the transpositional activity and H187I, H187V or H187L, more preferably H187V, and/or P247R; and/or K248A, or K248S; preferably K248R; or
   (48) K13A/K33A/T83N/R214D/K215A/E216V/N217Q increasing the transpositional activity and H187I, H187V or H187L, more preferably H187V, and/or P247R; and/or K248A, or K248S; preferably K248R.

In a preferred embodiment, the polypeptide of the first aspect comprises of consists of an amino acid of SEQ ID NO: 1 wherein the following amino acids have been substituted, which increase the transpositional activity: K14R, K33A, R115H, R214D/K215A/E216V/N217Q, M243H, and T314N and further comprising the following substitutions of the invention H187I, H187V or H187L, more preferably H187V, and/or P247R; and/or K248A, or K248S; preferably K248R. Particularly, preferred embodiments of the polypeptide of the first aspect of the invention have an amino acid sequence comprising or consisting of SEQ ID NO: 1 wherein the following amino acids have been substituted: substitutions:
K14R, K33A, R115H, R214D/K215A/E216V/N217Q, M243H, and T314N to increase the transpositional activity and H187V; or
K14R, K33A, R115H, R214D/K215A/E216V/N217Q, M243H, and T314N to increase the transpositional activity and P247R; or
K14R, K33A, R115H, R214D/K215A/E216V/N217Q, M243H, and T314N to increase the transpositional activity and K248R; or
K14R, K33A, R115H, R214D/K215A/E216V/N217Q, M243H, and T314N to increase the transpositional activity and P247R and K248R; preferably
K14R, K33A, R115H, R214D/K215A/E216V/N217Q, M243H, and T314N to increase the transpositional activity and H187V; or
K14R, K33A, R115H, R214D/K215A/E216V/N217Q, M243H, and T314N to increase the transpositional activity and P247R; or
K14R, K33A, R115H, R214D/K215A/E216V/N217Q, M243H, and T314N to increase the transpositional activity and K248R.

Particularly preferred transposases of the present invention that combine an increased transpositional activity with the properties of the substitutions of the present invention are the following (amino acid substitutions increasing the transpositional activity are highlighted by bold + underline and the amino acid substitutions of the invention are highlighted by bold, underline and italic):
H187V and K14R, K33A, R115H, R214D/K215A/E216V/N217Q, M243H, and T314N (SEQ ID NO: 20): 
MGKSKEISQD LRKRIVDLHK SGSSLGAISK RLAVPRSSVQ TIVRKYKHHG TTQPSYRSGR
RRVLSPRDER TLVRKVQINP RTTAKDLVKM LEETGTKVSI STVKRVLYRH NLKGHSARKK
PLLQNRHKKA RLRFATAHGD KDRTFWRNVL WSDETKIELF GHNDHRYVWR KKGEACKPKN
TIPTVKVGGG SIMLWGCFAA GGTGALHKID GIMDAVQYVD ILKQHLKTSV RKLKLGRKWV
FQHDNDPKHT SKVVAKWLKD NKVKVLEWPS QSPDLNPIEN LWAELKKRVR ARRPTNLTQL
HQLCQEEWAK IHPNYCGKLV EGYPKRLTQV KQFKGNATKY*
P247R and K14R, K33A, R115H, R214D/K215A/E216V/N217Q, M243H, and T314N (SEQ ID NO: 21): 
MGKSKEISQD LRKRIVDLHK SGSSLGAISK RLAVPRSSVQ TIVRKYKHHG TTQPSYRSGR
RRVLSPRDER TLVRKVQINP RTTAKDLVKM LEETGTKVSI STVKRVLYRH NLKGHSARKK
PLLQNRHKKA RLRFATAHGD KDRTFWRNVL WSDETKIELF GHNDHRYVWR KKGEACKPKN
TIPTVKHGGG SIMLWGCFAA GGTGALHKID GIMDAVQYVD ILKQHLKTSV RKLKLGRKWV
FQHDNDRKHT SKVVAKWLKD NKVKVLEWPS QSPDLNPIEN LWAELKKRVR ARRPTNLTQL
HQLCQEEWAK IHPNYCGKLV EGYPKRLTQV KQFKGNATKY*
K248R and K14R, K33A, R115H, R214D/K215A/E216V/N217Q, M243H, and T314N (SEQ ID NO: 22):

MGKSKEISQD LRKRIVDLHK SGSSLGAISK RLAVPRSSVQ TIVRKYKHHG TTQPSYRSGR
RRVLSPRDER TLVRKVQINP RTTAKDLVKM LEETGTKVSI STVKRVLYRH NLKGHSARKK
PLLQNRHKKA RLRFATAHGD KDRTFWRNVL WSDETKIELF GHNDHRYVWR KKGEACKPKN
TIPTVKHGGG SIMLWGCFAA GGTGALHKID GIMDAVQYVD ILKQHLKTSV RKLKLGRKWV
FQHDNDPRHT SKVVAKWLKD NKVKVLEWPS QSPDLNPIEN LWAELKKRVR ARRPTNLTQL
HQLCQEEWAK IHPNYCGKLV EGYPKRLTQV KQFKGNATKY*

The inventive polypeptides (transposase variants), have several advantages compared to approaches in the prior art with the most prominent exhibiting a remarkable gain of specificity of integration into the genome.

In a second aspect, the invention relates to a nucleic acid comprising a nucleic acid sequence encoding a polypeptide of the first aspect.

In one embodiment of the second aspect of the invention, the nucleic acid sequence is operably linked to at least one transcription control unit.

In one embodiment of the nucleic acid according to the invention the nucleic acid additionally comprises at least an open reading frame. In another embodiment, the nucleic acid additionally comprises at least a regulatory region of a gene. Preferably, the regulatory region is a transcriptional regulatory region, and more specifically the regulatory region is selected from the group consisting of a promoter, an enhancer, a silencer, a locus control region, and a border element. Nucleic acids according to the present invention typically comprise ribonucleic acids, including mRNA, DNA, cDNA, chromosomal DNA, extrachromosomal DNA, plasmid DNA, viral DNA or RNA, including also a recombinant viral vector. In one embodiment of the nucleic acid according to the invention the nucleic acid is DNA or RNA and in another preferred embodiment the nucleic acid is part of a plasmid or a recombinant viral vector. An inventive nucleic acid is preferably selected from any nucleic sequence encoding the amino acid sequence of the inventive polypeptide. Therefore, all nucleic acid variants coding for the above mentioned inventive mutated polypeptide variants including nucleic acid variants with varying nucleotide sequences due to the degeneration of the genetic code. In particular nucleotide sequences of nucleic acid variants which lead to an improved expression of the encoded fusion protein in a selected host organism, are preferred. Tables for appropriately adjusting a nucleic acid sequence to the host cell's specific transcription/translation machinery are known to a skilled person. In general, it is preferred to adapt the G/C-content of the nucleotide sequence to the specific host cell conditions. For expression in human cells an increase of the G/C content by at least 10%, more preferred at least 20%, 30%, 50%, 70% and even more preferred 90% of the maximum G/C content (coding for the respective inventive peptide variant) is preferred. Preparation and purification of such nucleic acids and/or derivatives are usually carried out by standard procedures.

These sequence variants preferably lead to inventive polypeptides or proteins selected from variants of the polypeptide with transpositional activity of the invention comprising an amino acid sequence according to SEQ ID NO: 1-17, preferably SEQ ID NO: 1, which have at least one amino acid substituted as compared to the native nucleic acid sequence of the corresponding polypeptide with transpositional activity. Therefore, inventive nucleic acid sequences code for modified (non-natural) variants of said polypeptide. Further, promoters or other expression control regions can be operably linked with the nucleic acid encoding the inventive polypeptide to regulate expression of the polypeptide/protein in a quantitative or in a tissue-specific manner.

In a third aspect, the invention relates to a vector comprising the nucleic acid molecule of the second aspect as described above. As already mentioned above the nucleic acid encoding the inventive polypeptide may be RNA or DNA. Similarly, either the inventive nucleic acid encoding the inventive polypeptide or the transposon can be a linear fragment or a circularized, isolated fragment or be inserted into a vector, preferably as a plasmid or as recombinant viral DNA.

In a fourth aspect, the invention relates to a cell comprising the nucleic acid of the second aspect or the vector of the third aspect.

In one embodiment the cell is from an animal, preferably a vertebrate, which is preferably selected from the group consisting of a fish, a bird, or a mammal, preferably a mammal,

In a fifth aspect, the invention relates to an *in vitro* method of integrating an exogenous nucleic acid into the genome of a cell, the method comprising the steps of:
a) providing an isolated cell;
b) providing in the cell a polypeptide of the invention; and
c) providing in the cell a nucleic acid comprising the exogenous nucleic acid or a vector comprising the same.

In one embodiment of the *in vitro* method,
(a) the polypeptide is provided to the cell by introducing the nucleic acid, or the vector of the invention into the cell; and/or
(b) the exogenous nucleic acid is comprised within the nucleic acid or the nucleic acid comprising the exogenous nucleic acids or a vector comprising the same is provided separately to the cell.

In one embodiment the nucleic acid of the invention, the vector of the invention and/or the exogenous nucleic acid is provided in the cell using a method selected from the group consisting of: electroporation, microinjection, lipofection.

The cell is from an animal, preferably a vertebrate, which is preferably selected from the group consisting of a fish, a bird, or a mammal, preferably a mammal.

In a sixth aspect, the invention relates to an *in vivo* method of integrating an exogenous nucleic acid into the genome of a cell of a subject, the method comprising the step of administering the polypeptide, the nucleic acid, or the vector of any of the afore mentioned aspects of the invention and a nucleic acid comprising the exogenous nucleic acid or a vector comprising said nucleic acid to the subject.

In one embodiment the exogenous nucleic acid is comprised in the nucleic acid or the vector of the invention or is administered separately.

In one embodiment the nucleic acid, or the vector of the invention or the exogenous nucleic acid is provided in the cell using a method selected from the group consisting of: electroporation, microinjection, lipoprotein particles, virus-like particles.

The exogenous nucleic acid may be DNA or RNA.

In another aspect, the invention relates to the mentioned polypeptide, nucleic acid, vector, or cell for use in medicine, in particular in gene therapy.

In one embodiment the gene therapy includes but is not limited to autologous or heterologous T cell therapy, gene therapy targeting any cell type in the blood, hematopoietic stem cell therapy, liver gene therapy, gene therapy of the central nervous system, eye gene therapy, muscle gene therapy, skin gene therapy.

In general the therapeutic applications of this invention may be manifold and thus polypeptide, the nucleic acid, the vector, the cell and especially the methods of integrating an exogenous nucleic acid into the genome of a cell according to the invention may also find use in therapeutic applications, in which the aforementioned are employed to stably integrate a therapeutic nucleic acid ("nucleic acid of therapeutic interest"), e.g. gene (nucleic acid of therapeutic interest), into the genome of a target cell, i.e. gene therapy applications. This may also be of interest for vaccination therapy for the integration of antigens into antigen presenting cells, e.g. specific tumor antigens, e.g. MAGE-1, for tumor vaccination or pathological antigens for the treatment of infectious diseases derived from pathogens, e.g. leprosy, tetanus, Whooping Cough, Typhoid Fever, Paratyphoid Fever, Cholera, Plague, Tuberculosis, Meningitis, Bacterial Pneumonia, Anthrax, Botulism, Bacterial Dysentry, Diarrhoea, Food Poisoning, Syphilis, Gasteroenteritis, Trench Fever, Influenza, Scarlet Fever, Diphtheria, Gonorrhoea, Toxic Shock Syndrome, Lyme Disease, Typhus Fever, Listeriosis, Peptic Ulcers, and Legionnaires' Disease; for the treatment of viral infections resulting in e.g. Acquired Immunodeficiency Syndrome, Adenoviridae Infections, Alphavirus Infections, Arbovirus Infections, Borne Disease, Bunyaviridae infections, Caliciviridae Infections, Chickenpox, Condyloma Acuminata, Coronaviridae Infections, Coxsackievirus Infections, Cytomegalovirus Infections, Dengue, DNA Virus Infections, Ecthyma, Contagious, Encephalitis, Arbovirus, Epstein-Barr Virus Infections, Erythema Infectiosum, Hantavirus Infections, Hemorrhagic Fevers, Viral, Hepatitis, Viral, Human, Herpes Simplex, Herpes Zoster, Herpes Zoster Oticus, Herpesviridae Infections, Infectious Mononucleosis, Influenza in Birds, Influenza, Human, Lassa Fever, Measles, Molluscum Contagiosum, Mumps, Paramyxoviridae Infections, Phlebotomus Fever, Polyomavirus Infections, Rabies, Respiratory Syncytial Virus Infections, Rift Valley Fever, RNA Virus Infections, Rubella, Slow Virus Diseases, Smallpox, Subacute Sclerosing Panencephalitis, Tumor Virus Infections, Warts, West Nile Fever, Virus Diseases, Yellow Fever; for the treatment of protozoological infections resulting in e.g. malaria. The subject methods may be used to deliver a wide variety of therapeutic nucleic acids. Therapeutic nucleic acids of interest include genes that replace defective genes in the target host cell, such as those responsible for genetic defect based diseased conditions; genes which have therapeutic utility in the treatment of cancer; and the like.

In a further aspect, the invention relates to a pharmaceutical composition comprising the mentioned polypeptide, nucleic acid, vector or cell and a pharmaceutically acceptable carrier, adjuvant or vehicle.

The pharmaceutical composition may further comprise one or more carriers and/or excipients, all of which are preferably pharmaceutically acceptable. According to the invention, a pharmaceutical composition contains an effective amount of the active agents, e.g., the polypeptide, nucleic acid, vector, or cell described herein, to generate the desired reaction or the desired effect. A pharmaceutical composition in accordance with the present invention is preferably sterile. Pharmaceutical compositions can be provided in a uniform dosage form and may be prepared in a manner known per se. A pharmaceutical composition in accordance with the present invention may, e.g., be in the form of a solution or suspension.

Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. The pharmaceutical compositions of the present invention may be administered orally, parenterally, by inhalation spray, topically, rectally, nasally, buccally, vaginally or via an implanted reservoir. The term parenteral as used herein includes subcutaneous, intravenous, intramuscular, intra-articular, intra-synovial, intrasternal, intrathecal, intrahepatic, intralesional and intracranial injection or infusion techniques. Preferably, the pharmaceutical compositions are administered orally, intraperitoneally or intravenously. Sterile injectable forms of the pharmaceutical compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

The inventive pharmaceutical composition is preferably suitable for the treatment of diseases, particular diseases caused by gene defects such as cystic fibrosis, hypercholesterolemia, hemophilia, e.g. A, B, C or XIII, immune deficiencies including HIV, Huntington disease, α-anti-Trypsin deficiency, as well as cancer selected from colon cancer, melanomas, kidney cancer, lymphoma, acute myeloid leukemia (AML), acute lymphoid leukemia (ALL), chronic myeloid leukemia (CML), chronic lymphocytic leukemia (CLL), gastrointestinal tumors, lung cancer, gliomas, thyroid cancer, mamma carcinomas, prostate tumors, hepatomas, diverse virus-induced tumors such as e.g. papilloma virus induced carcinomas (e.g. cervix carcinoma), adeno carcinomas, herpes virus induced tumors (e.g. Burkitt's lymphoma, EBV induced B cell lymphoma), Hepatitis B induced tumors (Hepato cell carcinomas), HTLV-1 und HTLV-2 induced lymphoma, akustikus neurinoma, lung cancer, pharyngeal cancer, anal carcinoma, glioblastoma, lymphoma, rectum carcinoma, astrocytoma, brain tumors, stomach cancer, retinoblastoma, basalioma, brain metastases, medullo blastoma, vaginal cancer, pancreatic cancer, testis cancer, melanoma, bladder cancer, Hodgkin syndrome, meningeoma, Schneeberger's disease, bronchial carcinoma, pituitary cancer, mycosis fungoides, gullet cancer, breast cancer, neurinoma, spinalioma, Burkitt's lymphoma, lyryngeal cancer, thymoma, corpus carcinoma, bone cancer, non-Hodgkin lymphoma, urethra cancer, CUP-syndrome, oligodendroglioma, vulva cancer, intestinal cancer, oesphagus carcinoma, small intestine tumors, craniopharyngeoma, ovarial carcinoma, ovarian cancer, liver cancer, leukemia, or cancers of the skin or the eye; etc..

There are a variety of alternative techniques and procedures available to those of skill in the art which would similarly permit one to successfully practice the intended invention.

The invention also pertains to the following items:
1. A polypeptide with transpositional activity comprising or consisting of a variant of a naturally occurring transposase with the following secondary structural elements: α1 helix-α2 helix-β1 sheet-β2 sheet-β3 sheet-β4 sheet-β5 sheet-α3 helix-β6 sheet-η1-α4 helix-η2-α5 helix-α6 helix-α7 helix-α8 helix
   wherein
   (i) a naturally occurring H in the loop connecting the β3 sheet and the β4 sheet is substituted with V, A, N, C, Q, G, I, L, M, F, P, R, S, T, W, K or Y, preferably with V or P, or
      a naturally occurring F in the loop connecting the β3 sheet and the β4 sheet is substituted with V, A, N, C, Q, G, I, L, M, P, R, S, T, W, K, H or Y, preferably with V or P, or
      a naturally occurring Y in the loop connecting the β3 sheet and the β4 sheet is substituted with V, A, N, C, Q, G, I, L, M, F, P, R, S, T, W, H or K, preferably with V or P, or
      a naturally occurring L in the loop connecting the β3 sheet and the β4 sheet is substituted with V, A, N, C, Q, G, I, M, F, P, R, S, T, W, H, Y or K, preferably with V or P, or
      a naturally occurring S in the loop connecting the β3 sheet and the β4 sheet is substituted with V, A, N, C, Q, G, I, L, M, F, P, R, T, W, H, Y or K, preferably with V or P;
         and/or
   (ii) at least one naturally occurring Q, P, S, or T in η1 connecting the β6 sheet with α4 helix is substituted with R, S or A, preferably with R;
      and/or
   (iii) at least one naturally occurring K, I, S, T, V, P or C in η1 connecting the β6 sheet with α4 helix is preferably substituted with R;
   wherein the variant of the naturally occurring transposase comprises at least one of the substations (i), (ii) or (iii).
2. The polypeptide of item 1, wherein the naturally occurring transposase is selected from the group consisting of Sleeping Beauty (SEQ ID NO: 1), Tdrl (SEQ ID NO: 2), ZB (SEQ ID NO: 3), FP (SEQ ID NO: 4), Passport (SEQ ID NO: 5), TCB2 (SEQ ID NO: 6), S (SEQ ID NO: 7), Quetzal (SEQ ID NO: 8), Paris (SEQ ID NO: 9), Tc1 (SEQ ID NO: 10), Minos (SEQ ID NO: 11), Uhu (SEQ ID NO: 12), Bari (SEQ ID NO: 13), Tc3 (SEQ ID NO: 14), Impala (SEQ ID NO: 15), Himar (SEQ ID NO: 16), or Mos1 (SEQ ID NO: 17), preferably the transposase is SB (SEQ ID NO: 1).
3. The polypeptide of item 1 or 2 comprising:
   (i) a first amino acid stretch:
      X₁X₂X₃Y₁X₄X₅GX₆ (SEQ ID NO: 18),
      X₁ is T, F, P, or R,
      X₂ is I, T, N, R, K, Q, or V,
      X₃ is K, N, L, R or Q, preferably K,
      X₄ is G, N, P, A, or Q, preferably G,
      X₅ is G, K, A, or is absent, preferably X₅ is G,
      X₆ is S, or is absent, and
      Y₁ is F, H, Y, L or S in the wild-type protein and
         wherein when Y₁ is F in the naturally occurring transposase and is unsubstituted or is substituted with A, N, C, Q, G, I, L, M, P, R, S, T, V, W, K or Y, preferably with V or P;
         wherein when Y₁ is H in the naturally occurring transposase and is unsubstituted or is substituted with A, N, C, Q, G, I, L, M, F, P, R, S, T, V, W, K or Y, preferably with V or P;
         wherein when Y₁ is Y in the naturally occurring transposase and is unsubstituted or is substituted with A, N, C, Q, G, I, L, M, F, P, R, S, T, V, W, or K, preferably with V or P;
         wherein when Y₁ is L in the naturally occurring transposase and is unsubstituted or is substituted with A, N, C, Q, G, I, F, M, P, R, S, T, V, W, K or Y, preferably with V or P;
         wherein when Y₁ is S in the naturally occurring transposase and is unsubstituted or is substituted with A, N, C, Q, G, I, L, F, M, P, R, T, V, W, K or Y, preferably with V or P;
            and/or
   (ii) a second amino acid stretch:
      X₇X₈DX₉X₁₀Y₂X₁₁Y₃H (SEQ ID NO: 19), wherein
      X₇ is Q, L, M, H or Y, and
      X₈ is M, Q or H,
      X₉ is N, H or G
      X₁₀ is A, or D,
      X₁₁ is V, T or K, and
      Y₂ is Q, P, S, or T in the naturally occurring transposase and is unsubstituted or is substituted with R or A, preferably with R,
         and/or
      Y₃ is K, I, S, T, V, P or C in the naturally occurring transposase and is unsubstituted or is substituted with R, A, P, or Q, preferably with R,
   wherein at least one of Y₁, Y₂ and Y₃ is substituted.
4. The polypeptide of item 3,
   wherein
   X₁ is T,
   X₂ is V or I, preferably V,
   X₃ is K or Q, preferably K,
   X₄ is G,
   X₅ is G,
   X₆ is S, and
   Y₁ is H, F or Y in the wild-type protein and
   Y₁ is F, H, Y, L or S in the wild-type protein and
   wherein when Y₁ is F in the naturally occurring transposase and is unsubstituted or is substituted with A, N, C, Q, G, I, L, M, P, R, S, T, V, W, K or Y, preferably with V or P;
   wherein when Y₁ is H in the naturally occurring transposase and is unsubstituted or is substituted with A, N, C, Q, G, I, L, M, F, P, R, S, T, V, W, K or Y, preferably with V or P;
   wherein when Y₁ is Y in the naturally occurring transposase and is unsubstituted or is substituted with A, N, C, Q, G, I, L, M, F, P, R, S, T, V, W, or K, preferably with V or P;
   wherein when Y₁ is L in the naturally occurring transposase and is unsubstituted or is substituted with A, N, C, Q, G, I, F, M, P, R, S, T, V, W, K or Y, preferably with V or P;
   wherein when Y₁ is S in the naturally occurring transposase and is unsubstituted or is substituted with A, N, C, Q, G, I, L, F, M, P, R, T, V, W, K or Y, preferably with V or P;
      and/or
   X₇ is Q,
   X₈ is M, Q or H,
   X₉ is N, H, or G
   X₁₀ is A, or D,
   X₁₁ is T, V or K
   Y₂ is P in the naturally occurring transposase and is unsubstituted or has been substituted with R or A, preferably with R, and/or
   Y₃ is K in the naturally occurring transposase and is unsubstituted or has been substituted with R, A, P, or Q, preferably with R, and
   wherein at least one of Y₁, Y₂ and Y₃ is substituted.
5. The polypeptide of any of items 1 to 4, comprising SEQ ID NO: 1 or variants thereof with transpositional activity and at least 70% sequence identity to SEQ ID NO: 1 and wherein the SEQ ID NO: 1 or the variant thereof comprises a substitution that increases its transpositional activity, preferably one or more of the following substitutions:
   (i) K248R. H187A, H187N, H187C, H187Q, H187G, H187I, H187L, H187M, H187F, H187S, H187V, H187W, or H187Y, preferably H187I, H187V or H187L, more preferably H187V; and/or
   (ii) P247R; and/or
   (iii) K248A, or K248S; preferably K248R;
   or a substitution at a corresponding position of the variant thereof.
6. The polypeptide of any of items 2 to 5, further comprising at least one of the following substitutions or groups of substitutions:
   (1) K14R, K13D, K13A, K30R, K33A, T83A, I100L, R115H, R143L, R147E, A205K/H207V/K208R/D210E, H207V/K208R/D210E, R214D/K215A/E216V/N217Q; M243Q, E267D, T314N, and/or G317E;
   (2) K14R//R214D/K215A/E216V/N217Q;
   (3) K33A/R115H/R214D/K215A/E216V/N217Q/M243H;
   (4) K14R/K30R/A205K/H207V/K208R/D210E/R214D/K215A/E216V/N217Q/M243H;
   (5) K13D/K33A/T83N/H207V/K208R/D210E/M243Q;
   (6) K13A/K33A/R214D/K215A/E216V/N217Q;
   (7) K33A/T83N/R214D/K215NE216V/N217QHG317E;
   (8) K14R/T83A/M243Q;
   (9) K14R/T83A/I100L/M243Q;
   (10) K14R/T83A/R143L/M243Q;
   (11) K14R/T83A/R147E/M243Q;
   (12) K14R/T83A/M243Q/E267D;
   (13) K14R/T83A/M243Q/T314N;
   (14) K14R/K30R/I110L/A205K/H207V/K208R/D210E/R214D/K215A/ E216V/N217Q/M243H;
   (15) K14R/K30R/R143L/A205K/H207V/K208R/D210E/R214D/K215A/ E216V/N217Q//M243H;
   (16) K14R/K30R/R147E/A205K/H207V/K208R/D210E/R214D/K215A/ E216V/N217Q/M243H;
   (17) K14R/K30R/A205K/H207V/K208R/D210E/R214D/K215A/ E216V/N217Q//M243H/E267D;
   (18) K14R/K30R/A205K/H207V/K208R/D210E/R214D/K215A/ E216V/N217Q/M243H/T314N;
   (19) K14R/K30R/A205K/H207V/K208R/D210E/R214D/K215A/ E216V/N217Q//M243H/G317E;
   (20) K14R/K33A/R115H/R214D/K215A/E216V/N217Q/M243H;
   (21) K14R/K30R/R147E/A205K/H207V/K208R/D210E/R214D/K215A/ E216V/N217Q//M243H/T314N;
   (22) K14R/K30R/R143U/A205K/H207V/K208R/D210E/R214D/K215A/ E216V/N217Q//M243H/E267D;
   (23) K14R/K30R/R143L/A205K/H207V/K208R/D210E/R214D/K215A/ E216V/N217Q//M243H/T314N;
   (24) K14R/K30R/R143L/A205K/H207V/K208R/D210E/R214D/K215A/ E216V/N217Q//M243H/G317E;
   (25) K14R/K33A/R115H/R143L/R214D/K215A/E216V/N217Q/M243H;
   (26) K14R/K33A/R115H/R147E//R214D/K215A/E216V/N217Q/M243H;
   (27) K14R/K33A/R115H/R214D/K215A/E216V/N217Q//M243H/E267D;
   (28) K14R/K33A/R115H/R214D/K215A/E216V/N217Q//M243H/T314N;
   (29) K14R/K33A/R115H/R214D/K215A/E216V/N217Q//M243H/G317E;
   (30) K14R/T83A/M243Q/G317E; or
   (31) K13A/K33A/T83N/R214D/K215A/E216V/N217Q;
   or at least one of the following substitution(s) or groups of substitutions at corresponding position(s) of the variant thereof.
7. A nucleic acid comprising a nucleic acid sequence encoding a polypeptide of any of items 1 to 6.
8. The nucleic acid of item 7, wherein the nucleic acid sequence is operably linked to at least one transcription control unit.
9. A vector comprising the nucleic acid molecule of item 7 or 8.
10. Cell comprising the nucleic acid of item 7 or 8 or the vector of item 9.
11. An *in vitro* method of integrating an exogenous nucleic acid into the genome of a cell, the method comprising the steps of:
   a) providing an isolated cell;
   b) providing in the cell a polypeptide of any of items 1 to 6; and
   c) providing in the cell a nucleic acid comprising the exogenous nucleic acid or a vector comprising the same.
12. The *in vitro* method according to item 10, wherein
   (a) the polypeptide is provided to the cell by introducing the nucleic acid of item 7 or 8 or the vector of item 9 into the cell; and/or
   (b) the exogenous nucleic acid is comprised within the nucleic acid or the nucleic acid comprising the exogenous nucleic acids or a vector comprising the same is provided separately to the cell.
13. The *in vitro* method according to any of items 11 or 12, wherein the nucleic acid of item 7 or 8, the vector of item 9 and/or the exogenous nucleic acid is provided in the cell using a method selected from the group consisting of: electroporation, microinjection, lipofection.
14. An *in vivo* method of integrating an exogenous nucleic acid into the genome of a cell of a subject, the method comprising the step of administering the polypeptide of any of items 1 to 6, the nucleic acid of item 7 or 8, or the vector of item 9 and a nucleic acid comprising the exogenous nucleic acid or a vector comprising said nucleic acid to the subject.
15. The *in vivo* method according to item 14, wherein the exogenous nucleic acid is comprised in the nucleic acid of item 7 or 8 or the vector of item 9 or is administered separately.
16. The *in vivo* method according to any of items 14 or 15, wherein the nucleic acid of item 6 or 7, the vector of item 8 or the exogenous nucleic acid is provided in the cell using a method selected from the group consisting of: electroporation, microinjection, lipoprotein particles, virus-like particles.
17. A polypeptide of items 1 to 6, a nucleic acid of item 7 or 8, a vector of item 9 or a cell of item 9 for use in medicine.
18. A polypeptide of items 1 to 6, a nucleic acid of item 7 or 8, a vector of item 9 or a cell of item 10 for use in gene therapy, including but not limited to autologous or heterologous T cell therapy, gene therapy targeting any cell type in the blood, hematopoietic stem cell therapy, liver gene therapy, gene therapy of the central nervous system, eye gene therapy, muscle gene therapy, skin gene therapy.
19. Pharmaceutical composition comprising a polypeptide of items 1 to 6, a nucleic acid of item 7 or 8, a vector of item 9 or a cell of item 10 and a pharmaceutically acceptable carrier, adjuvant or vehicle.

### Examples

### Example 1: Saturation mutagenesis of H187, P247 and K248 in the Sleeping Beauty transposase identifies mutants displaying altered efficiencies of transposition

In order to assess the relative effects of single amino acid replacements at positions 187, 247 and 248 on transposition, the SB100X transposase was subjected to saturation mutagenesis by incorporating all possible amino acids by site-directed PCR mutagenesis. All constructs encoding the mutant SB100X transposases showed protein expression levels comparable to that of SB100X by Western blot analysis.

Next we evaluated the mutants for their transposition activities relative to SB100X by applying a cell-based transposition assay in human cells that was finetuned to obtain a single transposon integration per cell. Briefly, a donor plasmid carrying a puromycin (puro) resistance gene-marked transposon was cotransfected together with a helper plasmid encoding either SB100X, the inactive E279D transposase (D3) or a mutant variant of the SB100X transposase.

In contrast to the H187 mutations, the vast majority of the P247 mutations was either completely inactive or displayed a severe reduction in both overall transposition (Fig. 3B) and excision (data not shown) activities. P247A was the most active mutant with 109% transposition activity relative to SB100X. Of note, the relative transposition activity of P247A exceeded the calculated relative excision activity. This discrepancy between excision- and integration capacities is likely due to the fact that both values were determined by two independent assays. The second most active mutant was P247S which retained 73% of transposon integration activity relative to SB100X, followed by P247R (27%, *p*=0.017), P247C (7%, p=0.002) and P247K (3%, *p*=0.003) **(****Fig. 3B****).**

Similar to P247, the amino acid exchanges at position 248 completely abolished transposition in almost all of the nineteen mutants **(****Fig. 3C****).** K248R, by far, was the most active mutant with 77% integration activity relative to SB100X. Only three additional K248 mutants, including K248C (4%, p=0.003), K248I (4%, *p*=0.003) and K248V (9%, *p*=0.003), displayed transposition activities at extremely low levels relative to SB100X. As described recently, some of the K248 mutants displayed a segregation of transposon excision and integration activities (data not shown).

### Example 2: Altered target site preferences of H187, P247 and K248 transposase mutants

In order to investigate if the mutations have an impact on target site selection, we used some of the SB mutants described above for the generation of transposon insertion site libraries in human HepG2 cells, and compared both the local attributes as well as genome-wide distributions of these insertion sites to those generated by SB100X. For position 187 we selected 6 mutants (H187R, H187E, H187S, H187T, H187V, H187P) with a marked impact on transposition efficiency **(****Fig. 3A****),** whereas for positions 247 and 248 we selected all mutants that displayed measurable transposition activity (P247A, P247R, P247C, P247S, P247K and K248C, K248I, K248V, K248R, respectively) **(****Fig. 3B** and **C**). Transposon integration sites were visualized by SeqLogo analysis that not only reports a consensus sequence, but also delivers information on overall sequence conservation (measured in bits) and relative frequencies of nucleotides in each position (measured as height of symbols which are ordered by their relative frequency within the logo). The analysis revealed that the highly preferred TA target site dinucleotides are embedded in A/T-rich DNA for all mutants, as noted previously for SB **(****Fig. 4****).** SB100X transposase prefers the 8-bp palindromic AT repeat sequence ATATATAT centered on the actual TA target dinucleotide. Because SB almost exclusively integrates into TA, the central TA approaches the maximal value of 2 bits in the logo. The SB100X reference logo also displays a relatively strong overall conservation of the A base in the -3 position and the matching T in the +3 position of the consensus with an overall score of 0.6 bits **(****Fig. 4****).** These integration preferences appear similar for all of the transposase mutants **(****Fig. 4** and **data not shown);** however, there are notable differences. First, the P247S and P247R mutants display slight deviations in their consensus target sites [AGATATCT for P247S and ATTATATAAT for P247 **(****Fig. 4****);** note that the palindromic nature of the consensus is maintained]. Second, the conservation of the alternating AT bases upstream and downstream of the 8-bp consensus becomes more pronounced with the mutants H187P, H187V and K248R, manifesting itself as "shoulders" flanking the 8-bp consensus **(****Fig. 4****).** Finally, conservation of the A in the -3 position and the T in the +3 position of the consensus is dramatically increased to ∼1.5 bits with the H187P, H187V and K248R mutants **(****Fig. 4****).** These findings indicate that amino acid replacements in position 187, 247 and 248 of the SB transposase indeed have an impact on the target site selection properties of the respective mutant transposases, and that the most significant alteration is a more pronounced overall preference for A/T-richness of the tDNA and stronger conservation of palindromic bases within the consensus sequences. The data thus shows that some of the mutants became more constrained in their target site selection properties. This is remarkably revealed by analyzing the overall frequencies of transposon insertions into the 8-bp ATATATAT sequence.

Although SB100X and the P247S and P247R mutants target this particular sequence only 2-3% of the time, some other mutants display significantly higher frequencies of integration into this motif (18% for H187P, 21% for H187V and 39% for K248R, **Fig. 4**). In sum, some amino acid substitutions in positions 187, 247 and 248 of the SB transposase result in a phenotype of highly preferential integration into AT-repeats.

### Example 3: Altered genome-wide distribution of insertions catalyzed by H187, P247 and K248 transposase mutants

We determined the relative frequencies of integration into genomic features including genic and non-genic regions, cancer genes, exons, introns, 5'- and 3'-UTRs and sequences flanking genes upstream and downstream within 10-kb windows, relative to a computer-generated random data set. We not only compared insertions within these genomic features generated by SB100X and its mutants, but also involved MLV gammaretroviral and HIV lentiviral integration sites in the analysis. Both of these viral systems are popular vectors in gene therapy. As established previously, SB transposon insertions show only a minor bias toward genes and their flanking regions, in contrast to MLV and HIV insertions that are enriched in loci flanking transcriptional start sites (TSS) and within actively transcribed genes, respectively **(****Fig. 5****).** Out of these three gene vector systems the overall insertion frequencies of SB are the closest to an expected random distribution **(****Fig. 5****).**

Next, we selected a small subset of our mutants; namely the P247R, H187V and K248R mutants, and addressed if these mutants generate a recognizably different genome-wide distribution profile than that of SB 100X. The data presented in **Fig. 5A** are revealing. Especially the H187V and K248R mutants show a significant depletion in insertion into genes. The most significant differences are within 5'- and 3'-UTRs as well as exons including coding exons. Importantly, transposon integration by H187V and K248R within these genomic regions is not only depleted in comparison to SB 100X, but also in comparison to the random dataset. The overall percentages of insertions into these genomic regions are presented in **Fig. 5E** The most dramatic change in integration frequency is a 4-fold drop seen with K248R in comparison to SB100X (*p*<0.001) in coding exons **(****Fig. 5A****).** Beyond driving integration away from exons, a preference of integrating into AT-repeats by our mutants also implies that these insertions might be enriched in repetitive DNA. Indeed, a dramatic enrichment in simple repeats is detected for integrations catalyzed by H187V and K248R; the enrichment in this compartment of the genome is ∼12-fold over the random dataset (*p*<0.001) and ∼4-fold over SB100X (*p*<0.001) by H187V, and ∼21-fold over the random dataset (*p*<0.001) and ∼7-fold over SB100X (*p*<0.001) by K248R **(****Fig. 5C****).** Consistent with a preference of integration into ATATATAT, the H187V variant displays a 24-fold and ∼5-fold enrichment over random and SB100X (*p*<0.001), respectively, in TA-rich simple repeats, whereas K248R-mediated insertions are ∼43-fold and ∼8-fold enriched over random and SB100X (*p*<0.001), respectively, in TA-rich simple repeats **(****Fig. 5D****).**

Next, we profiled insertions generated by SB100X and the P247R, H187V and K248R mutants in functional genomic segments. These segments are defined by cooccurring epigenetic signal patterns, which are clustered together computationally to comprise various functional partitions of the human genome. We used 25-state chromatin models of human HepG2 cells. As above, we included MLV and HIV insertions in the analysis. First, in line with previous observations, SB transposon insertions show only a minor bias towards promoters, TSSs, enhancers and transcriptional regulatory regions with an open chromatin structure, in contrast to MLV and HIV insertions that display the highest enrichment in promoter regions including TSSs and transcribed regions, respectively **(****Fig. 5B****).** Second, a dramatic depletion in enhancers, promoters including TSSs and open regulatory regions is seen for integrations catalyzed by H187V and K248R; the most significant changes being a ∼50-fold, ∼3-fold and ∼5-fold depletion over MLV, SB100X (*p*<0.001) and the random dataset, respectively, by H187V in promoters including TSSs, and a ∼14-fold, 4-foldand ∼3-fold depletion over MLV, SB100X (*p*<0.001) and the random dataset, respectively, by K248R in enhancers **(****Fig. 5B****).** Collectively, the data show that the P247R, H187V and K248R mutants de-target a significant fraction of transposon integrations away from exons as well as transcriptional regulatory regions including promoters and enhancers.

### Example 4: Enrichment of insertions into genomic safe harbors by the H187V, P247R and K248R transposase mutants

Integration of therapeutic gene constructs into safe sites in the human genome would prevent insertional mutagenesis and associated risks of oncogenesis in gene therapy. Genomic "safe harbors" (GSHs) are regions of the human genome that are able to accommodate the predictable expression of newly integrated DNA without adverse effects on the host cell or organism. GSHs can be bioinformatically allocated to chromosomal sites or regions if they satisfy the following criteria: (i) no overlap with transcription units, (ii) distance of at least 50 kb from the 5'-end of any gene, iii) at least 300 kb distance to cancer related genes and (iv) microRNA genes, and (v) regions outside of ultra-conserved elements (UCEs) 58, 68. We have previously established that the SB transposon system has a significantly more favorable insertion profile than MLV- and HIV-based viral integration systems with respect to frequencies of insertions into GSHs.

The data presented above establish that some of our SB transposase mutants significantly de-target insertions away from exons as well as transcriptional regulatory regions of genes, thereby inherently implying that a larger fraction of insertions catalyzed by these enzymes lands in GSHs. We analyzed our insertion site datasets with respect to the relative frequencies of integration into GSHs by the P247R, H187V and K248R transposase variants and, as above, we included MLV and HIV insertions in the analysis **(****Fig. 6). Fig. 6A** displays three clusters in the dendogram: the first represented by MLV and HIV, the second by SB100X, P247R and H187V and the third by K248R and random. There is a gradual shift towards a random-like distribution of insertions by the SB100X transposase and its mutants. For example, only 17% of HIV insertions fall outside of genes, whereas this value is 41% for SB 100X, 42% for P247R, 44% for H187V, 46% for K248R and 49% for the random dataset **(****Fig. 6A****).** In line with these observations, our analyses revealed increasing frequencies of insertions into GSHs by simultaneously applying all five GSH criteria. Out of the mutants tested, the K248R transposase variant approached random the closest: overall, 29% of all K248R insertions and 34% of all random insertions fall in a GSH **(****Fig. 6B****).** Collectively, our analysis predicts 1) a safer, thus favorable transgene insertion profile for the SB system over MLV-, and HIV-based vectors and 2) an enhanced safety of the P247R, H187V and K248R transposase variants in therapeutic gene transfer in human cells.

### Example 5: The H187V and K248R transposase variants avoid nucleosomal DNA for integration

High-density integration profiling of the *Hermes* transposon in yeast revealed a strong association of integration sites with nucleosome-free chromatin. In addition, recent evidence indicates that Tc1/*mariner* transposons preferentially integrate at linker regions between nucleosomes. We mapped our insertion datasets with respect to nucleosome occupancy as determined by MNase-Seq data. As seen previously, transposon insertions mediated by SB100X are underrepresented in nucleosomal DNA **(****Fig. 7****).** Remarkably, out of the three transposase mutants tested, the H187V and K248R variants displayed a drastic drop in association of transposon integration sites and nucleosome occupancy, not only as compared to the random control, but also to the SB100X dataset **(****Fig. 7****).** Thus, the H187V and K248R mutations dramatically intensify a phenotype of the SB transposase; namely, avoidance of nucleosomal DNA for transposon integration.

### Example 6: Molecular features associated with preferential genomic target sites of H187, P247 and K248 transposase mutants

The above data establish that the H187V and K248R transposase mutants detarget exons and transcriptional regulatory regions of genes and avoid nucleosomal DNA for integration. However, these data do not necessarily shed light on causative relationships between these independent observations. For example, exons tend to be associated with nucleosomes; thus, depletion of integrations in exonic sequences by H187V and K248R may be a mere reflection of these transposase variants avoiding nucleosomal DNA. However, transcriptional regulatory regions including enhancers and TSSs are clearly depleted in nucleosomes; nonetheless we found that H187V and K248R detarget these genomic regions, suggesting that nucleosome occupancy in itself cannot sufficiently explain why H187Vand K248R integrations are depleted in both exons as well as regulatory regions. Because the genomic segments are defined by chromatin marks, the question arises whether it is the local chromatin structure or the underlying primary DNA sequence that modulates integration frequencies in these segments. Consistent with previous findings 27, the SB100X transposase catalyzes an almost random insertion profile in human cells with a slight bias for euchromatin marks (including H3K4me1, H3K27ac, H3K36me3 and H3K29me2) (Fig. 8A). We detect a significant (p<0.001) depletion in chromatin marks by the H187V and K248R variants as compared to SB100X insertions (Fig. 8C); however, the depletion is not restricted to euchromatin marks. Instead, H187V and K248R insertions are generally depleted in histone modifications, regardless whether they mark transcriptionally active or repressed chromatin (Fig. 8A); thus, we consider a differential interaction of the H187V and K248R transposase variants with chromatin as compared to SB100X unlikely. The above findings led us to hypothesize that the primary determinant of preferred genomic targets by the H187, P247 and K248 transposase mutants is DNA sequence composition. Exons, in particular coding exons, are relatively poor in TA dinucleotides (Fig. 8B), which are the preferred target sites for SB transposition. Having seen a pronounced preference by some of our mutants, especially by H187V and K248R, for the ATATATAT sequence motif implies that de-targeting of exon sequences by this mutant is driven by the rare occurrence of this sequence motif in exonic sequences. Indeed, ATATATAT is severely underrepresented in exons, especially so in coding exons (Fig. 8B), thereby suggesting that primary DNA sequence composition is the major determinant of driving H187V and K248R insertions away from exonic sequences. Similarly, open regulatory regions, TSSs and enhancers are relatively TA-poor in comparison to the average base composition of the human genome (Fig. 8C). As seen for coding exons, the ATATATAT sequence motif, into which the H187V and K248R mutants preferentially integrate, is severely underrepresented in these three genomic segments (Fig. 8C). The data argue that the major

## Claims

1. A polypeptide with transpositional activity comprising or consisting of a variant of a naturally occurring transposase with the following secondary structural elements:
α1 helix-α2 helix-β1 sheet-β2 sheet-β3 sheet-β4 sheet-β5 sheet-α3 helix-β6 sheet-η1-α4 helix-η2-α5 helix-α6 helix-α7 helix-α8 helix
wherein
(i) a naturally occurring H in the loop connecting the β3 sheet and the β4 sheet is substituted with V, A, N, C, Q, G, I, L, M, F, P, R, S, T, W, K or Y, preferably with V or P, or
a naturally occurring F in the loop connecting the β3 sheet and the β4 sheet is substituted with V, A, N, C, Q, G, I, L, M, P, R, S, T, W, K, H or Y, preferably with V or P, or
a naturally occurring Y in the loop connecting the β3 sheet and the β4 sheet is substituted with V, A, N, C, Q, G, I, L, M, F, P, R, S, T, W, H or K, preferably with V or P, or
a naturally occurring L in the loop connecting the β3 sheet and the β4 sheet is substituted with V, A, N, C, Q, G, I, M, F, P, R, S, T, W, H, Y or K, preferably with V or P, or
a naturally occurring S in the loop connecting the β3 sheet and the β4 sheet is substituted with V, A, N, C, Q, G, I, L, M, F, P, R, T, W, H, Y or K, preferably with V or P;
and/or
(ii) at least one naturally occurring Q, P, S, or T in η1 connecting the β6 sheet with α4 helix is substituted with R, S or A, preferably with R;
and/or
(iii) at least one naturally occurring K, I, S, T, V, P or C in η1 connecting the β6 sheet with α4 helix is preferably substituted with R;
wherein the variant of the naturally occurring transposase comprises at least one of the substations (i), (ii) or (iii).

2. The polypeptide of claim 1, wherein the naturally occurring transposase is selected from the group consisting of Sleeping Beauty (SEQ ID NO: 1), Tdrl (SEQ ID NO: 2), ZB (SEQ ID NO: 3), FP (SEQ ID NO: 4), Passport (SEQ ID NO: 5), TCB2 (SEQ ID NO: 6), S (SEQ ID NO: 7), Quetzal (SEQ ID NO: 8), Paris (SEQ ID NO: 9), Tc1 (SEQ ID NO: 10), Minos (SEQ ID NO: 11), Uhu (SEQ ID NO: 12), Bari (SEQ ID NO: 13), Tc3 (SEQ ID NO: 14), Impala (SEQ ID NO: 15), Himar (SEQ ID NO: 16), or Mos1 (SEQ ID NO: 17), preferably the transposase is SB (SEQ ID NO: 1).

3. The polypeptide of claim 1 or 2 comprising:
(i) a first amino acid stretch:
X₁X₂X₃Y₁X₄X₅GX₆ (SEQ ID NO: 18),
X₁ is T, F, P, or R,
X₂ is I, T, N, R, K, Q, or V,
X₃ is K, N, L, R or Q, preferably K,
X₄ is G, N, P, A, or Q, preferably G,
X₅ is G, K, A, or is absent, preferably X₅ is G,
X₆ is S, or is absent, and
Y₁ is F, H, Y, L or S in the wild-type protein and
wherein when Y₁ is F in the naturally occurring transposase and is unsubstituted or is substituted with A, N, C, Q, G, I, L, M, P, R, S, T, V, W, K or Y, preferably with V or P;
wherein when Y₁ is H in the naturally occurring transposase and is unsubstituted or is substituted with A, N, C, Q, G, I, L, M, F, P, R, S, T, V, W, K or Y, preferably with V or P;
wherein when Y₁ is Y in the naturally occurring transposase and is unsubstituted or is substituted with A, N, C, Q, G, I, L, M, F, P, R, S, T, V, W, or K, preferably with V or P;
wherein when Y₁ is L in the naturally occurring transposase and is unsubstituted or is substituted with A, N, C, Q, G, I, F, M, P, R, S, T, V, W, K or Y, preferably with V or P;
wherein when Y₁ is S in the naturally occurring transposase and is unsubstituted or is substituted with A, N, C, Q, G, I, L, F, M, P, R, T, V, W, K or Y, preferably with V or P;
and/or
(ii) a second amino acid stretch:
X₇X₈DX₉X₁₀Y₂X₁₁Y₃H (SEQ ID NO: 19), wherein
X₇ is Q, L, M, H or Y, and
X₈ is M, Q or H,
X₉ is N, H or G
X₁₀ is A, or D,
X₁₁ is V, T or K, and
Y₂ is Q, P, S, or T in the naturally occurring transposase and is unsubstituted or is substituted with R or A, preferably with R,
and/or
Y₃ is K, I, S, T, V, P or C in the naturally occurring transposase and is unsubstituted or is substituted with R, A, P, or Q, preferably with R,
wherein at least one of Y₁, Y₂ and Y₃ is substituted.

4. The polypeptide of claim 3,
wherein
X₁ is T,
X₂ is V or I, preferably V,
X₃ is K or Q, preferably K,
X₄ is G,
X₅ is G,
X₆ is S, and
Y₁ is H, F or Y in the wild-type protein and
Y₁ is F, H, Y, L or S in the wild-type protein and
wherein when Y₁ is F in the naturally occurring transposase and is unsubstituted or is substituted with A, N, C, Q, G, I, L, M, P, R, S, T, V, W, K or Y, preferably with V or P; wherein when Y₁ is H in the naturally occurring transposase and is unsubstituted or is substituted with A, N, C, Q, G, I, L, M, F, P, R, S, T, V, W, K or Y, preferably with V or P; wherein when Y₁ is Y in the naturally occurring transposase and is unsubstituted or is substituted with A, N, C, Q, G, I, L, M, F, P, R, S, T, V, W, or K, preferably with V or P; wherein when Y₁ is L in the naturally occurring transposase and is unsubstituted or is substituted with A, N, C, Q, G, I, F, M, P, R, S, T, V, W, K or Y, preferably with V or P; wherein when Y₁ is S in the naturally occurring transposase and is unsubstituted or is substituted with A, N, C, Q, G, I, L, F, M, P, R, T, V, W, K or Y, preferably with V or P;
and/or
X₇ is Q,
X₈ is M, Q or H,
X₉ is N, H, or G
X₁₀ is A, or D,
X₁₁ is T, V or K
Y₂ is P in the naturally occurring transposase and is unsubstituted or has been substituted with R or A, preferably with R, and/or
Y₃ is K in the naturally occurring transposase and is unsubstituted or has been substituted with R, A, P, or Q, preferably with R, and
wherein at least one of Y₁, Y₂ and Y₃ is substituted.

5. The polypeptide of any of claims 1 to 4, comprising SEQ ID NO: 1 or variants thereof with transpositional activity and at least 70% sequence identity to SEQ ID NO: 1 and wherein the SEQ ID NO: 1 or the variant thereof comprises a substitution that increases its transpositional activity, preferably one or more of the following substitutions:
(i) K248R. H187A, H187N, H187C, H187Q, H187G, H187I, H187L, H187M, H187F, H187S, H187V, H187W, or H187Y, preferably H187I, H187V or H187L, more preferably H187V; and/or
(ii) P247R; and/or
(iii) K248A, or K248S; preferably K248R;
or a substitution at a corresponding position of the variant thereof.

6. The polypeptide of any of claims 2 to 5, further comprising at least one of the following substitutions or groups of substitutions:
(1) K14R, K13D, K13A, K30R, K33A, T83A, I100L, R115H, R143L, R147E, A205K/H207V/K208R/D210E, H207V/K208R/D210E, R214D/K215A/E216V/N217Q; M243Q, E267D, T314N, and/or G317E;
(2) K14R//R214D/K215A/E216V/N217Q;
(3) K33A/R115H/R214D/K215A/E216V/N217Q/M243H;
(4) K14R/K30R/A205K/H207V/K208R/D210E/R214D/K215A/E216V/N217Q/M243H;
(5) K13D/K33A/T83N/H207V/K208R/D210E/M243Q;
(6) K13A/K33A/R214D/K215A/E216V/N217Q;
(7) K33A/T83N/R214D/K215NE216V/N217QHG317E;
(8) K14R/T83A/M243Q;
(9) K14R/T83A/I100L/M243Q;
(10) K14R/T83A/R143L/M243Q;
(11) K14R/T83A/R147E/M243Q;
(12) K14R/T83A/M243Q/E267D;
(13) K14R/T83A/M243Q/T314N;
(14) K14R/K30R/I110L/A205K/H207V/K208R/D210E/R214D/K215A/ E216V/N217Q/M243H;
(15) K14R/K30R/R143L/A205K/H207V/K208R/D210E/R214D/K215A/ E216V/N217Q//M243H;
(16) K14R/K30R/R147E/A205K/H207V/K208R/D210E/R214D/K215A/ E216V/N217Q/M243H;
(17) K14R/K30R/A205K/H207V/K208R/D210E/R214D/K215A/ E216V/N217Q//M243H/E267D;
(18) K14R/K30R/A205K/H207V/K208R/D210E/R214D/K215A/ E216V/N217Q/M243H/T314N;
(19) K14R/K30R/A205K/H207V/K208R/D210E/R214D/K215A/ E216V/N217Q//M243H/G317E;
(20) K14R/K33A/R115H/R214D/K215A/E216V/N217Q/M243H;
(21) K14R/K30R/R147E/A205K/H207V/K208R/D210E/R214D/K215A/ E216V/N217Q//M243H/T314N;
(22) K14R/K30R/R143U/A205K/H207V/K208R/D210E/R214D/K215A/ E216V/N217Q//M243H/E267D;
(23) K14R/K30R/R143L/A205K/H207V/K208R/D210E/R214D/K215A/ E216V/N217Q//M243H/T314N;
(24) K14R/K30R/R143L/A205K/H207V/K208R/D210E/R214D/K215A/ E216V/N217Q//M243H/G317E;
(25) K14R/K33A/R115H/R143L/R214D/K215A/E216V/N217Q/M243H;
(26) K14R/K33A/R115H/R147E//R214D/K215A/E216V/N217Q/M243H;
(27) K14R/K33A/R115H/R214D/K215A/E216V/N217Q//M243H/E267D;
(28) K14R/K33A/R115H/R214D/K215A/E216V/N217Q//M243H/T314N;
(29) K14R/K33A/R115H/R214D/K215A/E216V/N217Q//M243H/G317E;
(30) K14R/T83A/M243Q/G317E; or
(31) K13A/K33A/T83N/R214D/K215A/E216V/N217Q;
or at least one of the following substitution(s) or groups of substitutions at corresponding position(s) of the variant thereof.

7. A nucleic acid comprising a nucleic acid sequence encoding a polypeptide of any of claims 1 to 6, wherein optionally the nucleic acid sequence is operably linked to at least one transcription control unit.

8. A vector comprising the nucleic acid molecule of claim 7.

9. Cell comprising the nucleic acid of claim 7 9 or the vector of claim 8.

10. An *in vitro* method of integrating an exogenous nucleic acid into the genome of a cell, the method comprising the steps of:
a) providing an isolated cell;
b) providing in the cell a polypeptide of any of claims 1 to 6; and
c) providing in the cell a nucleic acid comprising the exogenous nucleic acid or a vector comprising the same.

11. The *in vitro* method according to claim 10, wherein
(a) the polypeptide is provided to the cell by introducing the nucleic acid of claim 7 or 8 or the vector of claim 9 into the cell; and/or
(b) the exogenous nucleic acid is comprised within the nucleic acid or the nucleic acid comprising the exogenous nucleic acids or a vector comprising the same is provided separately to the cell.

12. An *in vivo* method of integrating an exogenous nucleic acid into the genome of a cell of a subject, the method comprising the step of administering the polypeptide of any of claims 1 to 6, the nucleic acid of claim 7, or the vector of claim 8 and a nucleic acid comprising the exogenous nucleic acid or a vector comprising said nucleic acid to the subject.

13. A polypeptide of claims 1 to 6, a nucleic acid of claim 7, a vector of claim 8 or a cell of claim 9 for use in medicine.

14. A polypeptide of claims 1 to 6, a nucleic acid of claim 7, a vector of claim 8 or a cell of claim 9 for use in gene therapy, including but not limited to autologous or heterologous T cell therapy, gene therapy targeting any cell type in the blood, hematopoietic stem cell therapy, liver gene therapy, gene therapy of the central nervous system, eye gene therapy, muscle gene therapy, skin gene therapy.

15. Pharmaceutical composition comprising a polypeptide of claims 1 to 6, a nucleic acid of claim 7 or 8, a vector of claim 9 or a cell of claim 10 and a pharmaceutically acceptable carrier, adjuvant or vehicle.
